(19) Europäisches Patentamt
European Patent Office
Office européen des brevets

(11) **EP 4 098 260 A1**

(12) **EUROPEAN PATENT APPLICATION**
published in accordance with Art. 153(4) EPC

(43) Date of publication:
**07.12.2022  Bulletin 2022/49**

(21) Application number: **21747930.2**

(22) Date of filing: **26.01.2021**

(51) International Patent Classification (IPC):
*A61K 31/4439* (2006.01)    *A61K 9/14* (2006.01)
*A61K 9/20* (2006.01)    *A61K 9/28* (2006.01)
*A61P 1/04* (2006.01)

(52) Cooperative Patent Classification (CPC):
**A61K 9/14; A61K 9/20; A61K 9/28; A61K 31/4439;
A61P 1/04**

(86) International application number:
**PCT/JP2021/002521**

(87) International publication number:
**WO 2021/153525 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **27.01.2020  JP 2020011208**

(71) Applicant: **TOWA PHARMACEUTICAL CO., LTD.
Kadoma-shi,
Osaka 571-8580 (JP)**

(72) Inventors:
• **HIRAISHI Keisuke
  Kadoma-shi, Osaka 571-8580 (JP)**

• **YOSHIMURA Takuma
  Kadoma-shi, Osaka 571-8580 (JP)**
• **HONJO Tatsuya
  Kadoma-shi, Osaka 571-8580 (JP)**
• **SAEKI Isamu
  Kadoma-shi, Osaka 571-8580 (JP)**
• **OKUDA Yutaka
  Kadoma-shi, Osaka 571-8580 (JP)**

(74) Representative: **Grünecker Patent- und
Rechtsanwälte
PartG mbB
Leopoldstraße 4
80802 München (DE)**

(54) **ESOMEPRAZOLE ORAL PREPARATION AND METHOD FOR PRODUCING SAME**

(57)    A spherical core containing an esomeprazole compound as an active ingredient and having a median particle diameter ($d_{50}$) of 50 $\mu$m or more is coated with a coat containing a controlled release layer to prepare a controlled release granule containing a controlled release granule with a median particle diameter ($d_{50}$) of 350 $\mu$m or less. The spherical core may contain the esomeprazole compound at a proportion of 50% by mass or more relative to the spherical core. The spherical core may be a spherical particle containing the esomeprazole compound as a drug. The spherical core has a median particle diameter ($d_{50}$) of 50 $\mu$m or more. The spherical core may have a sphericity of 0.6 or more. The coat may be in a multilayer free from an esomeprazole compound as an active ingredient. The controlled release layer may be an enteric coating layer. The proportion of the coat may be 80% by mass or more relative to the whole amount of the preparation. The oral preparation may be an orally disintegrating tablet containing a disintegrator in addition to the controlled release granule. The oral preparation has an excellent controlled release ability and an excellent formulation designability such as miniaturization.

[Fig. 5]

50x  200 μm  WD: 9.6mm  5kV  2019/03/07 09:26:58 S

EP 4 098 260 A1

**Description**

TECHNICAL FIELD

The present invention relates to an oral preparation containing esomeprazole acting as a proton pump inhibitor, as an active ingredient, and method for producing the oral preparation.

BACKGROUND ART

Esomeprazole acting as a proton pump inhibitor is an S-enantiomer [or (-)-enantiomer] obtained by optical resolution of omeprazole, which is a racemate. Esomeprazole is weak against an acid, and esomeprazole is thus to be coated with an enteric polymer in an oral preparation.

Japanese Patent No. 3665334 (JP 3665334 B, Patent Document 1) discloses, as a stable oral enteric-coated formulation, a formulation which consists of a core material containing a magnesium salt of omeprazole, a subcoating layer coating the core material, and an enteric coating layer coating the subcoating layer. In Examples of this document, the subcoating layer is formed by spraying a hydroxypropylmethylcellulose aqueous solution on a spherical granule containing omeprazole magnesium, mannitol, a microcrystalline cellulose and anhydrous lactose as main ingredients and having a particle diameter of 0.5 to 1.5 mm, and the enteric coating layer is formed by spraying an aqueous solution containing a methacrylic acid copolymer and a polyethylene glycol on the subcoating layer.

Japanese Patent No. 4649001 (JP 4649001 B, Patent Document 2) discloses an oral preparation having a core material, a separating layer, and an enteric coating layer, wherein the separating layer and the enteric coating layer are disposed on the core material; the core material contains at least one active ingredient and a binder, the active ingredient is selected from omeprazole, an alkaline salt thereof, esomeprazole, and an alkaline salt thereof, and a constitutional component of the binder and/or the separating layer is a low viscosity hydroxypropylmethylcellulose having a specific cloud point.

Japanese Patent No. 3350054 (JP 3350054 B, Patent Document 3) discloses an oral pharmaceutical preparation which contains a pellet or granule having an enteric coating layer and having a gastric acid resistance that is not significantly affected in compression into a tablet; the oral pharmaceutical preparation includes a core material containing at least one active substance selected from omeprazole, an alkaline salt thereof, esomeprazole, and an alkaline salt thereof, wherein the core material is coated with at least one enteric coating layer, at least one layer of the enteric coating layer(s) contains a plasticizer in an amount of 20 to 50% by mass relative to the enteric coating layer polymer, and the plasticizer is selected from triacetin, a citric acid ester, a phthalic acid ester, dibutyl sebacate, cetyl alcohol, a polyethylene glycol, and a polysorbate. In Examples of this document, a pellet having an enteric coating layer is mixed with a granule containing a diluent or excipient such as a microcrystalline cellulose, and the mixture is compressed by a tableting machine to produce a tablet. As the pellet having the enteric coating layer, for example, prepared are: a pellet obtained by coating a core material with an enteric coating layer, wherein the core material is obtained by spraying a sugar sphere seed with an aqueous suspension of esomeprazole and a povidone, and the enteric coating layer consists of a methacrylic acid copolymer, triethyl citrate, and a talc; and a pellet obtained by coating a core material with a separating layer and an enteric coating layer, wherein the core material is obtained by spraying a sugar sphere seed with an aqueous suspension of esomeprazole magnesium and a hydroxypropylmethylcellulose, and the separating layer consists of a hydroxypropylcellulose, a talc, and magnesium stearate, and the enteric coating layer consists of a methacrylic acid copolymer, triethyl citrate, a mono- and di-glyceride, and Polysorbate 80.

Japanese Patent Application Laid-Open Publication No. 2018-118936 (JP 2018-118936 A, Patent Document 4) discloses, as an enteric solid preparation of which an enteric layer is preventable from breakage, an orally disintegrating tablet which contains an enteric granule consisting of a proton pump inhibitor as a main active ingredient and having a multilayer structure, wherein an enteric layer and/or a layer outside an enteric layer is coated with a coating composition containing mannitol and a polyethylene glycol. In Examples of this document, a spherical crystalline cellulose is coated with a drug layer containing esomeprazole magnesium hydrate, a hypromellose, a low substituted hydroxypropylcellulose, and Polysorbate 80, and the coated spherical crystalline cellulose is further coated with an intermediate layer, an elution control layer, an enteric layer, and a breakage prevention layer formed with the coating composition to prepare a granule having an average particle diameter of 300 μm, and then the granule is tableted to produce a tablet having a diameter of 10 mm.

CITATION LIST

PATENT LITERATURE

Patent Document 1: JP 3665334 B
Patent Document 2: JP 4649001 B
Patent Document 3: JP 3350054 B
Patent Document 4: JP 2018-118936 A

## SUMMARY OF INVENTION

### TECHNICAL PROBLEM

For the formulation of Patent Document 1, the formulation increases in size due to a large particle diameter of the spherical granule coated with the enteric coating layer, and has a limited formulation design. Furthermore, in a case where the formulation is used as an orally disintegrating tablet, the formulation has a feeling of roughness in the mouth and has a reduced comfortability of taking.

Patent Document 2 does not describe the sizes or shapes of the oral preparation and the core material. Thus, depending on the shape of the core material, the enteric coating layer is non-uniform (or ununiform) and has a low acid resistance.

For the preparations of Patent Documents 3 and 4, a spherical particle such as a sugar sphere seed or a spherical crystalline cellulose is provided as a core (a spherical diluent core), and the core is coated with a coating layer containing a main active ingredient and an enteric coating layer to prepare a spherical preparation having a uniform enteric coating layer. The preparations of Patent Documents 3 and 4 contain the spherical diluent core and thus unfortunately increase in size. Moreover, as mentioned above, in an orally disintegrating tablet, since the size of a coated granule of a drug affects the comfortability of taking, it is desired to reduce the size of the granule. However, reducing the amount of the coating component easily breaks the enteric coating layer by tableting, which decreases the acid resistance of the tablet. In the orally disintegrating tablet, there is a trade-off relationship between improvement of the comfortability of taking by miniaturization (or downsizing) and improvement of the acid resistance, and it is difficult to achieve both improvements. Thus, the use of the spherical diluent core for obtaining a spherical preparation may be disadvantageous in formulation design. In addition to the use of the spherical diluent core, the core is to be coated with the layer containing a main active ingredient, which complicates a production process.

It is therefore an object of the present invention to provide an esomeprazole oral preparation having an excellent controlled release ability and an excellent formulation designability such as miniaturization, and a method for producing the preparation.

Another object of the present invention is to provide an esomeprazole oral preparation having an excellent acid resistance and an excellent comfortability of taking, and a method for producing the preparation.

It is still another object of the present invention to provide an esomeprazole oral preparation being efficiently producible, having less feeling of roughness in the mouth even when used as an orally disintegrating tablet, and having no or less decrease in acid resistance after tableting, and a method for producing the preparation.

### SOLUTION TO PROBLEM

The inventors of the present invention made intensive studies to achieve the above objects and found that a spherical core containing an esomeprazole compound as an active ingredient and having a median particle diameter ($d_{50}$) of not less than 50 $\mu$m is coated with a coat containing a controlled release layer to prepare a controlled release granule having a median particle diameter ($d_{50}$) of not more than 350 $\mu$m, and the controlled release granule enables an improved formulation designability such as miniaturization while maintaining an excellent controlled release ability. The present invention was accomplished based on the above findings.

That is, the present invention includes an oral preparation containing a controlled release granule having a median particle diameter (or a median particle size) ($d_{50}$) of not more than 350 $\mu$m; the controlled release granule contains (or is formed with) a spherical core having a median particle diameter (or a median particle size) ($d_{50}$) of not less than 50 $\mu$m and containing an esomeprazole compound, and a coat coating the spherical core and containing a controlled release layer. The spherical core (or spherical core portion) may contain the esomeprazole compound at a proportion of not less than 50% by mass relative to the spherical core. The spherical core may be a spherical particle of the esomeprazole compound. The spherical core may have a sphericity of not less than 0.6. The coat (or coating portion) may be in the form of a multilayer (or may contain a plurality of layers) free from an esomeprazole compound. The coat may further contain a shielding layer interposed between the spherical core and the controlled release layer. The coat may further contain an intermediate layer interposed between the spherical core and the shielding layer. The controlled release layer may be an enteric coating layer. The proportion of the coat may be not less than 80% by mass relative to the whole amount of the preparation. The oral preparation may be in the form of a tablet. For example, the oral preparation may further contain a disintegrator and may be in the form of an orally disintegrating tablet.

The present invention also includes a method for producing the oral preparation, the method including: a spherical-core-forming step of forming a spherical core containing an esomeprazole compound, and a coating step of coating the resulting spherical core with a coat to give a controlled release granule. In the spherical-core-forming step, a spherulite may be precipitated from a supersaturated solution of the esomeprazole compound having a supersaturation larger than a critical supersaturation to form a spherical core.

The present invention also includes a method for improving a controlled release ability and a formulation designability of an oral preparation, the method including: adjusting a median particle diameter ($d_{50}$) of a controlled release granule contained in an oral preparation to not more than 350 $\mu$m, and preparing the controlled release granule containing a spherical core and a coat coating the spherical core, the spherical core having a median particle diameter ($d_{50}$) of not less than 50 $\mu$m and containing an esomeprazole compound, and the coat containing a controlled release layer.

The present invention also includes a method of use of a controlled release granule for improving a controlled release ability and a formulation designability of an oral preparation; the controlled release granule contains (or is formed with) a spherical core having a median particle diameter ($d_{50}$) of not less than 50 $\mu$m and containing an esomeprazole compound, and a coat coating the spherical core and containing a controlled release layer, and has a median particle diameter ($d_{50}$) of not more than 350 $\mu$m.

ADVANTAGEOUS EFFECTS OF INVENTION

According to the present invention, the median particle diameter ($d_{50}$) of the controlled release granule is adjusted to not more than 350 $\mu$m, wherein the controlled release granule contains the spherical core coated with the coat, the spherical core contains the esomeprazole compound as an active ingredient and has a median particle diameter ($d_{50}$) of not less than 50 $\mu$m, and the coat contains the controlled release layer. The controlled release granule can improve the formulation designability such as miniaturization while maintaining an excellent controlled release ability. In particular, the formation of the enteric coating layer as the controlled release layer can improve the acid resistance and the comfortability of taking. Moreover, the spherical core containing the esomeprazole compound eliminates the need for a spherical diluent core, and the controlled release granule has a high productivity. Furthermore, since the enteric coating layer has a thin and uniform thickness, the oral preparation has less feeling of roughness in the mouth even when used as an orally disintegrating tablet, and eliminates decrease in acid resistance after tableting. Thus, the oral preparation can effectively express the pharmacological activity of the esomeprazole compound even when orally administered.

BRIEF DESCRIPTION OF DRAWINGS

[Fig. 1] Fig. 1 is a diagram showing a method of calculating a sphericity.
[Fig. 2] Fig. 2 is a graph showing a relationship between an RMSE value and a sphericity.
[Fig. 3] Fig. 3 is a scanning electron microscope (SEM) photograph of esomeprazole magnesium hydrate used in Example 1.
[Fig. 4] Fig. 4 is a scanning electron microscope (SEM) photograph of a spherical core of esomeprazole magnesium hydrate obtained in Example 1.
[Fig. 5] Fig. 5 is a scanning electron microscope (SEM) photograph of a controlled release granule obtained in Example 1.
[Fig. 6] Fig. 6 is a scanning electron microscope (SEM) photograph of an extruded granule of esomeprazole magnesium hydrate obtained in Comparative Example 1.
[Fig. 7] Fig. 7 is a scanning electron microscope (SEM) photograph of an intermediate-layer-coated granule obtained in Comparative Example 1.

DESCRIPTION OF EMBODIMENTS

[Spherical core]

An oral preparation of the present disclosure contains a controlled release granule having a median particle diameter (or a median particle size) ($d_{50}$) of not more than 350 $\mu$m, and the granule contains a spherical core in a central portion thereof.

The spherical core (or spherical core portion) contains an esomeprazole compound as an active ingredient. The esomeprazole compound in the spherical core may be at least one member selected from the group consisting of esomeprazole, a salt of esomeprazole, a solvate of esomeprazole, and a solvate of a salt of esomeprazole.

The salt may be any pharmaceutically acceptable salt. The salt is usually a salt with a base. The base may include, for example, an inorganic base [for example, ammonia; an alkali metal (such as lithium, sodium, and potassium), an alkaline earth metal (such as calcium and magnesium), and other metals (such as zinc and aluminum)], and an organic base (for example, an alkylamine such as methylamine and triethylamine; a polyamine; an alkanolamine such as ethanolamine and triethanolamine; and a cyclic amine such as morpholine, piperazine, pyrrolidine, and picoline). These bases may be used alone or in combination of two or more. Among these bases, the metal such as the alkali metal and the alkaline earth metal is preferred, and the alkaline earth metal such as magnesium is particularly preferred.

A solvent forming the solvate may include, for example, water, an alcohol (for example, a $C_{1-4}$alkanol such as methanol, ethanol, 1-propanol, and 2-propanol), a ketone (for example, acetone, methyl ethyl ketone, methyl isopropyl ketone, and methyl isobutyl ketone), a nitrile (for example, acetonitrile and propionitrile), an ester (for example, ethyl acetate and isopropyl acetate), an ether (for example, diethyl ether and t-butyl methyl ether), an aliphatic hydrocarbon (for example, n-pentane, n-hexane, cyclohexane, n-heptane, and isooctane), an aromatic hydrocarbon (for example, toluene), an amide (for example, N,N-dimethylformamide and N,N-dimethylacetamide), and a sulfoxide (such as dimethyl sulfoxide). These solvents may be used alone or in combination of two or more. As the solvate, a hydrate is preferred, and a trihydrate is particularly preferred.

In particular, the esomeprazole compound is preferably in the form of a salt and/or a hydrate of esomeprazole and is usually in the form of a hydrate of an esomeprazole salt (such as esomeprazole magnesium trihydrate).

In this description and claims, esomeprazole, an esomeprazole salt (or a salt of esomeprazole), an esomeprazole solvate (or a solvate of esomeprazole), and an esomeprazole salt solvate (or a solvate of an esomeprazole salt) are collectively referred to as an esomeprazole compound.

The proportion of the esomeprazole compound in the spherical core may be not less than 50% by mass, and is, for example, not less than 70% by mass, preferably not less than 80% by mass, more preferably not less than 90% by mass, and most preferably 100% by mass (the spherical core substantially contains only the esomeprazole compound). In a case where the proportion of the esomeprazole compound is excessively low, the controlled release granule may have a large particle diameter, resulting in a low formulation designability.

The spherical core may further contain an additive including, for example, the after-mentioned binder, diluent, and lubricant. The total proportion of the additive(s) in the spherical core is not more than 50% by mass, and is, for example, not more than 30% by mass, preferably not more than 20% by mass, more preferably not more than 10% by mass, and most preferably 0% by mass.

The esomeprazole compound, which is a main ingredient of the spherical core, may be a non-crystalline (or amorphous) body, and is preferably a crystalline body. The shape of the crystalline body may include, for example, a plate crystal, an acicular crystal, a column crystal, a hopper crystal, a symmetric dendrite crystal, an asymmetric dendrite crystal, and a spherulite. Among them, the spherulite is preferred from the viewpoint of formation of the spherical core with the esomeprazole compound alone and improvement of the formulation designability.

In this description and claims, the term "spherulite" means a crystalline body being a crystal aggregate (polycrystal), having a radial tissue (or structure) or a concentrically laminated tissue (or structure), and having a spherical outline. Whether the crystalline body of the esomeprazole compound is a spherulite or not can be determined by, for example, a method of observing the outline of the crystalline body with a scanning electron microscope (SEM) or a method of cutting the crystalline body and observing the internal structure thereof with an SEM.

The spherical core may be in any spherical shape. The spherical core may have a sphericity (a degree of true sphericity) of not less than 0.6 (for example, about 0.6 to 0.99) and, for example, has a sphericity (a degree of sphericity) of not less than 0.7 (for example, 0.7 to 0.98), preferably not less than 0.8, more preferably not less than 0.9, and most preferably not less than 0.95. In a case where the sphericity is excessively low, it may be difficult to form a smooth coat. In particular, the sphericity may be a sphericity of a spherulite of the esomeprazole compound.

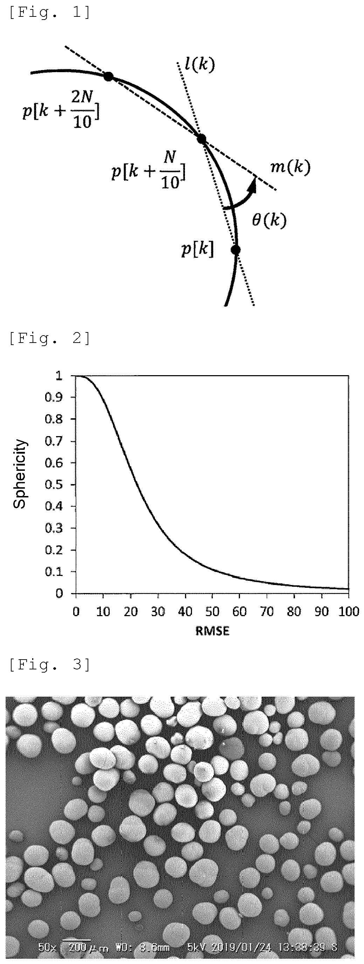

In this description and claims, the sphericity can be measured by the following method: in analyzing a particle image taken by an SEM with ImageJ, which is an image processing software developed at National Institutes of Health (NIH), acquire a group of coordinates (or a coordinate group) representing the positions of pixels on the outline (or contour) of the particle; let N be a length of the coordinate group (the number of pixels on the outline) and let p[i] ($1 \leq i \leq N$) be a coordinate of each pixel on the outline; let $\theta(k)$ ($-180° < \theta < 180°$) be an angle between a straight line l(k) and a straight line m(k), where the straight line l(k) is a line connecting two points p[k], p[k+N/D] and the straight line m(k) is a line connecting two points p[k+N/D], p[k+2N/D] ($1 \leq k \leq N$, $1 \leq D \leq N$), where D corresponds to the number of partitions (or divisions) of the outline, or D denotes a quotient produced by dividing N by D if N/D is not a natural number; and acquire N number of $\theta$ values by calculating the angle in k = 1 to N. The position of $\theta(k)$ is shown in Fig. 1.

The angle $\theta(k)$ can be calculated by, for example, the following equation:

[Math. 1]

$$\theta(k) = \arctan \frac{a(k) - b(k)}{1 + a(k)b(k)}$$

wherein a(k) represents a slope of the straight line l(k), and b(k) represents a slope of the straight line m(k).

When the particle is in a true spherical shape, $\theta(k) = 360/D$. As the shape of the particle is closer to a true spherical shape, $\theta(k)$ is closer to this value. The error between $\theta(k)$ and the true value 360/N is evaluated by the following equation for Root Mean Squared Error (RMSE):

[Math. 2]

$$\text{RMSE} = \sqrt{\frac{1}{N} \sum_{k=1}^{N} \left( \frac{360}{D} - \theta(k) \right)^2}$$

Using the RMSE value when D = 10 ($\theta(k)$ = 36°), the sphericity is defined by the following equation:

[Math. 3]

$$\text{Sphericity} = 1 - \frac{1}{1 + \exp\left(-6(\log(\text{RMSE}) - 1.3)\right)}$$

For a true spherical shape, the sphericity is 1. As the shape is closer to a true spherical shape, the sphericity is closer to 1. Meanwhile, an ellipse having an infinitely long axis as a major axis has a sphericity of zero. The sphericity is measured for five or more particles, and the average is determined. The average value is taken as a sphericity of a sample (see Figs. 1 and 2).

The spherical core has a median particle diameter (or a median particle size) ($d_{50}$) of not less than 50 $\mu$m, for example, 50 to 250 $\mu$m, preferably 100 to 200 $\mu$m, more preferably 120 to 180 $\mu$m, and most preferably 130 to 150 $\mu$m. In a case where the median particle diameter of the spherical core is excessively small, the esomeprazole compound may have a low efficacy. In a case where the spherical core is excessively large, the oral preparation may have a low formulation designability, and may have a low comfortability of taking depending on the application.

In this description and claims, the median particle diameter ($d_{50}$) can be measured by a particle size distribution measuring apparatus, and means a diameter corresponding to 50% from the smallest in a volume-based cumulative particle size (or particle diameter) distribution. In detail, the median particle diameter can be measured according to the method described in the after-mentioned Examples.

The particle size distribution (or particle diameter distribution) of the spherical core may have a sharpness index selected from a range of about 1 to 5. The sharpness index is, for example, 1 to 4, preferably 1 to 3, more preferably 1 to 2.5, even more preferably 1 to 2, and most preferably 1 to 1.5. The sharpness index indicates a uniformity of particle diameter. A particle size distribution having a sharpness index of 1 means a distribution having the most uniform particle diameter. An excessive large sharpness index may result in a low formulation designability.

In this description and claims, the sharpness index can be calculated from the values $d_{10}$, $d_{50}$, and $d_{90}$ measured by a particle size distribution measuring apparatus using the following equation:

$$\text{Sharpness Index} = [(d_{90}/d_{50}) + (d_{50}/d_{10})]/2$$

[Controlled release layer]

In the controlled release granule, the spherical core is coated with a coat (or a coating portion). The coat contains a controlled release layer. The controlled release layer is a layer capable of controlling release of the esomeprazole compound, which is an active ingredient (an active ingredient). The controlled release layer may be a sustained release layer, which controls a release speed (or rate), a transfer control layer, which controls transfer, or other layers. The controlled release layer is preferably an enteric coating layer, which is not dissolved in a gastric juice and is dissolved in an intestinal juice.

The enteric coating layer contains an enteric polymer. As the enteric polymer, a conventional enteric polymer may be used. The conventional enteric polymer may include, for example, a cellulose derivative such as a cellulose phthalate, a cellulose acetate phthalate, a hydroxypropylcellulose phthalate, a hydroxypropylmethylcellulose phthalate (HPMCF), and a hydroxypropylmethylcellulose acetate succinate; a (meth)acrylic polymer such as a methacrylic acid-ethyl acrylate copolymer (methacrylic acid copolymer LD), an ethyl acrylate-methyl methacrylate copolymer, a methacrylic acid-n-butyl acrylate copolymer, and a methacrylic acid-methyl methacrylate copolymer (methacrylic acid copolymer L, S); and a polyvinyl acetate phthalate. These enteric polymers may be used alone or in combination of two or more. Among them, preferred is the (meth)acrylic polymer such as the methacrylic acid copolymer LD and the ethyl acrylate-methyl methacrylate copolymer.

The proportion of the enteric polymer in the enteric coating layer may be not less than 10% by mass, and is, for example, 30 to 95% by mass, preferably 50 to 90% by mass, more preferably 60 to 85% by mass, even more preferably 70 to 80% by mass, and most preferably 75 to 80% by mass. In a case where the proportion of the enteric polymer is excessively low, the oral preparation may be low enteric or may have a low bioavailability.

The enteric coating layer may further contain a plasticizer. The plasticizer may include, for example, a hydrophilic plasticizer such as ethylene glycol, propylene glycol, and glycerin; and a lipophilic plasticizer such as an ethyl acrylate-methyl methacrylate copolymer, triacetin, triethyl citrate, diethyl phthalate, dioctyl adipate, lauric acid, stearyl alcohol, and cetanol. These plasticizers may be used alone or in combination of two or more. Among them, the lipophilic plasticizer such as triethyl citrate is preferred.

The proportion of the plasticizer relative to 100 parts by mass of the enteric polymer is, for example, 1 to 60 parts by mass, preferably 5 to 50 parts by mass, more preferably 8 to 40 parts by mass, even more preferably 10 to 30 parts by mass, and most preferably 10 to 20 parts by mass. In a case where the proportion of the plasticizer is excessively low, it may be difficult to form a smooth enteric coating layer. In a case where the proportion of the plasticizer is excessively high, the oral preparation may be low enteric.

The enteric coating layer may further contain a surfactant. The surfactant may include, for example, a macrogol (such as a polyethylene glycol having a weight-average molecular weight of 300 to 6000), a polyoxyethylene polyoxypropylene glycol (such as Pluronic and Poloxamer), a polysorbate (e.g., a polyoxyethylene sorbitan ester of a fatty acid, such as Polysorbate 80), a polyoxyethylene hydrogenated oil (such as a polyoxyethylene hydrogenated castor oil), a glycerol ester of a fatty acid (such as glyceryl monostearate), a sorbitan ester of a fatty acid (such as sorbitan monostearate and sorbitan monolaurate), a sucrose ester of a fatty acid (such as sucrose laurate), and a metal salt of a fatty acid (such as sodium lauryl sulfate). These surfactants may be used alone or in combination of two or more. Among them, the glycerol ester of a fatty acid and the polysorbate are preferred, and a glycerol ester of a mono-$C_{8-24}$ fatty acid, such as glyceryl monostearate, is particularly preferred.

The proportion of the surfactant relative to 100 parts by mass of the enteric polymer is, for example, 1 to 30 parts by mass, preferably 3 to 20 parts by mass, more preferably 5 to 15 parts by mass, and most preferably 7 to 10 parts by mass. In a case where the proportion of the surfactant is excessively low, it may be difficult to form a smooth enteric coating layer. In a case where the proportion of the surfactant is excessively high, the oral preparation may be low enteric.

The enteric coating layer may further contain a pH adjusting agent. The pH adjusting agent may include, for example, an inorganic acid (such as hydrochloric acid, sulfuric acid, and phosphoric acid), an organic acid (such as acetic acid and citric acid), an inorganic base (such as sodium hydroxide and sodium bicarbonate), and an organic base (such as an amine). These pH adjusting agents may be used alone or in combination of two or more. Among them, the organic acid or a hydrate thereof, such as citric acid hydrate, is preferred.

The proportion of the pH adjusting agent may suitably be selected according to a desired pH; the proportion of the pH adjusting agent relative to 100 parts by mass of the enteric polymer is, for example, 0.001 to 0.1 parts by mass, preferably 0.005 to 0.05 parts by mass, and more preferably 0.01 to 0.03 parts by mass.

The enteric coating layer may further contain a shielding agent (a first shielding agent) in addition to the enteric polymer. A shielding agent contained in the after-mentioned shielding layer can effectively protect the esomeprazole compound, which is an active ingredient; a shielding agent contained in the enteric coating layer can further improve such a shielding or protecting function.

The shielding agent for the controlled release layer may include, for example, a silicic acid compound (such as a talc, a light anhydrous silicic acid, a calcium silicate, a magnesium silicate, a synthetic aluminum silicate, and a magnesium aluminometasilicate), a metal oxide (such as magnesium oxide and titanium oxide), a carbonate (such as precipitated calcium carbonate and magnesium carbonate), a lactate (such as calcium lactate), a phosphate (such as anhydrous dibasic calcium phosphate and calcium monohydrogen phosphate), and a mineral (such as a bentonite, a synthetic hydrotalcite, and a kaolin). These shielding agents may be used alone or in combination of two or more. Among them, the silicic acid compound such as the talc and the metal oxide such as titanium oxide are preferred, and the talc is particularly preferred.

The proportion of the first shielding agent relative to 100 parts by mass of the enteric polymer is, for example, 1 to 100 parts by mass, preferably 10 to 50 parts by mass, and more preferably 20 to 40 parts by mass.

The enteric coating layer may further contain a lubricant (a first lubricant).

The first lubricant may include, for example, a fatty acid or a metal salt thereof (such as stearic acid, magnesium stearate, calcium stearate, sodium stearyl fumarate, and sodium cocoate), a silicon oxide (such as hydrated silicon dioxide and silicon dioxide), a polyorganosiloxane (such as a dimethylpolysiloxane), an oil and fat (such as a hydrogenated oil and a cacao butter), and a wax (such as a yellow beeswax, a white beeswax, a carnauba wax, a lanolin, a paraffin, and a petrolatum). These lubricants may be used alone or in combination of two or more. Among them, the fatty acid or the metal salt thereof and the silicon oxide are preferred, and the silicon oxide such as hydrated silicon dioxide is particularly preferred.

The proportion of the first lubricant relative to 100 parts by mass of the enteric polymer is, for example, 0.01 to 10 parts by mass, preferably 0.02 to 1 part by mass, and more preferably 0.03 to 0.1 parts by mass.

The enteric coating layer may further contain other additives including the after-mentioned binder, coloring agent, and/or diluent. In the enteric coating layer, the total proportion of other additives relative to 100 parts by mass of the enteric polymer is, for example, not more than 10 parts by mass, and is preferably not more than 5 parts by mass (for example, 0.1 to 5 parts by mass).

The controlled release layer may be in the form of a single layer (or may have a single-layered structure) or may be in the form of a multilayer (or may have a laminated structure). The controlled release layer in the form of a multilayer (or the controlled release layer formed with a plurality of layers) may have a laminated structure of a first enteric coating layer containing a surfactant and a second enteric coating layer free from a surfactant.

The controlled release layer (in particular, the enteric coating layer) has an average thickness (for a laminated structure, an average thickness of a total thickness) of, for example, 10 to 80 $\mu$m, preferably 15 to 70 $\mu$m, and more preferably 20 to 60 $\mu$m. In a case where the thickness of the controlled release layer is excessively small (or thin), the oral preparation may have a low controlled release ability. In a case where the thickness is excessively large (or thick), the oral preparation may have a low formulation designability.

In this description and claims, the average thickness of the controlled release layer can be calculated based on the material mass (or material amount used); the same applies to other layers. In a case where the controlled release layer is in the form of a multilayer, the above-mentioned average thickness means an average thickness of the total thickness of the multilayer; the same applies to other layers.

[Shielding layer]

The coat may further contain a shielding layer interposed between the spherical core and the controlled release layer. A component in the controlled release layer (in particular, the enteric polymer in the enteric coating layer) has a functional group such as carboxyl group, and the functional group easily reduces the action of the active ingredient. In the present disclosure, the interposition of the shielding layer between the controlled release layer and the spherical core can prevent the efficacy of the esomeprazole compound, which is an active ingredient in the spherical core, from being reduced due to the functional group.

The shielding layer contains a shielding agent (a second shielding agent). As the shielding agent, there may be used any shielding agent exemplified in the first shielding agent of the controlled release layer. The shielding agents may be used alone or in combination of two or more. Among the shielding agents, the silicic acid compound such as the talc and the metal oxide such as titanium oxide are preferred, and the talc is particularly preferred.

The proportion of the second shielding agent in the shielding layer may be not less than 10% by mass, and is, for example, about 20 to 90% by mass, preferably about 30 to 85% by mass, more preferably about 40 to 80% by mass, even more preferably about 50 to 70% by mass, and most preferably about 50 to 60% by mass. In a case where the proportion of the shielding agent is excessively low, the esomeprazole compound may have a low efficacy.

The shielding layer may further contain a binder (a first binder). The binder may include, for example, a synthetic polymer such as a polyvinylpyrrolidone compound (such as a povidone and a vinyl acetate-vinylpyrrolidone copolymer), a polyvinyl alcohol, a carboxyvinyl polymer, a polyacrylic polymer (such as a sodium polyacrylate and an acrylic acid copolymer), a polylactic acid, a polyethylene glycol, and a polyvinyl acetate; a cellulose ether such as a methylcellulose (MC), an ethylcellulose (EC), a carboxymethylcellulose (CMC), a carboxymethylethylcellulose (CMEC), a hydroxyethylcellulose, a hydroxypropylcellulose (HPC), and a hydroxypropylmethylcellulose (HPMC); and a cellulose ester such as a cellulose acetate. These binders may be used alone or in combination of two or more. Among them, a $C_{1-4}$alkylcellulose such as the ethylcellulose and a hydroxy$C_{2-4}$alkylcellulose such as the hydroxypropylcellulose are preferred; in order to improve an acid resistance of the tablet, a $C_{2-3}$alkylcellulose (in particular, the hydroxypropylcellulose and the ethylcellulose) is preferred, and the hydroxypropylcellulose is particularly preferred.

The hydroxypropylcellulose may use a commercially available product. The commercially available product may include, for example, product names "HPC-M (viscosity: 150 to 400 mPa·s)", "HPC-SL (viscosity: 3.0 to 5.9 mPa·s)", and "HPC-L (viscosity: 6.0 to 10.0 mPa·s)", all manufactured by Nippon Soda Co., Ltd. The viscosity of the hydroxypropylcellulose indicates a viscosity measured at a temperature of 20°C with a 2% aqueous solution.

The proportion of the first binder relative to 100 parts by mass of the second shielding agent is, for example, 10 to 100 parts by mass, preferably 20 to 60 parts by mass, more preferably 25 to 50 parts by mass, and most preferably 30 to 40 parts by mass. In a case where the proportion of the binder is excessively low, the shielding layer may have low mechanical characteristics. In a case where the proportion of the binder is excessively high, the oral preparation may have a low formulation designability.

The shielding layer may further contain a lubricant (a second lubricant). As the lubricant, there may be used any lubricant exemplified in the first lubricant of the controlled release layer. The lubricants may be used alone or in combination of two or more. Among the lubricants, the fatty acid or the metal salt thereof and the silicon oxide are preferred, and a metal salt of a $C_{8-24}$ fatty acid, such as magnesium stearate, is particularly preferred.

The proportion of the second lubricant relative to 100 parts by mass of the second shielding agent is, for example, 1 to 100 parts by mass, preferably 3 to 80 parts by mass, more preferably 5 to 70 parts by mass, even more preferably 10 to 60 parts by mass, and most preferably 40 to 55 parts by mass. In a case where the proportion of the lubricant is excessively low, it may be difficult to form a smooth shielding layer. In a case where the proportion of the lubricant is excessively high, the shielding layer may have low mechanical characteristics.

The shielding layer may further contain a coloring agent. The coloring agent may include, for example, yellow ferric oxide, red ferric oxide, Food Blue No. 1, Food Blue No. 2, Food Yellow No. 4, Food Yellow No. 5, Food Green No. 3, Food Red No. 2, Food Red No. 3, Food Red No. 102, Food Red No. 104, Food Red No. 105, Food Red No. 106, edible lake pigments, bengala, a turmeric extract, riboflavin, riboflavin sodium phosphate, a carotene solution, a tar color (or a coal-tar dye), and a caramel. These coloring agents may be used alone or in combination of two or more. Among them, yellow ferric oxide and red ferric oxide are preferred.

The proportion of the coloring agent relative to 100 parts by mass of the second shielding agent is, for example, 0.01 to 1 part by mass, preferably 0.05 to 0.5 parts by mass, and more preferably 0.1 to 0.3 parts by mass.

The shielding layer may further contain other additives including, for example, the above-mentioned plasticizer, surfactant, pH adjusting agent, and/or the after-mentioned diluent. In the shielding layer, the total proportion of other additives relative to 100 parts by mass of the shielding agent is, for example, not more than 10 parts by mass, and is preferably not more than 5 parts by mass (for example, 0.1 to 5 parts by mass).

The average thickness of the shielding layer (for a laminated structure, the average thickness of the total thickness) may be selected from a range of about 1 to 50 μm, and is, for example, 1 to 30 μm, preferably 2 to 20 μm, more preferably 3 to 15 μm, and most preferably 4 to 10 μm. In a case where the thickness of the shielding layer is excessively small (or thin), the esomeprazole compound may have a low efficacy. In a case where the thickness of the shielding layer is excessively large (or thick), the oral preparation may have a low formulation designability.

[Intermediate layer]

The coat may further contain an intermediate layer interposed between the shielding layer and the spherical core.

The intermediate layer contains a shielding agent (a third shielding agent). Thus, the intermediate layer may be referred to as a second shielding layer. As the third shielding agent, there may be used any shielding agent exemplified in the first shielding agent of the controlled release layer. The shielding agents may be used alone or in combination of two or more. Among the shielding agents, the talc and titanium oxide are preferred, and a combination of the talc and titanium oxide are particularly preferred.

The proportion of the third shielding agent in the intermediate layer may be not less than 30% by mass, and is, for example, about 30 to 95% by mass, preferably about 40 to 90% by mass, more preferably about 45 to 80% by mass, and most preferably about 50 to 60% by mass. In a case where the proportion of the third shielding agent is excessively low, the esomeprazole compound may have a low efficacy.

The intermediate layer may further contain a binder (a third binder). As the third binder, there may be used any binder exemplified in the first binder of the controlled release layer. The binders may be used alone or in combination of two or more. Among the binders, a hydroxy$C_{2-4}$alkylcellulose such as the hydroxypropylcellulose is preferred, and the hydroxypropylcellulose is particularly preferred.

The proportion of the third binder relative to 100 parts by mass of the third shielding agent is, for example, 1 to 50 parts by mass, preferably 3 to 40 parts by mass, more preferably 5 to 35 parts by mass, even more preferably 10 to 30 parts by mass, and most preferably 20 to 25 parts by mass. In a case where the proportion of the binder for the intermediate layer is excessively low, the shielding layer may have low mechanical characteristics. In a case where the proportion of the binder for the intermediate layer is excessively high, the oral preparation may have a low formulation designability.

The intermediate layer may further contain a lubricant (a third lubricant). As the third lubricant, there may be used any lubricant exemplified in the first lubricant of the controlled release layer. The lubricants may be used alone or in combination of two or more. Among the lubricants, the fatty acid or the metal salt thereof and the silicon oxide are preferred, and a metal salt of a $C_{8-24}$ fatty acid, such as magnesium stearate, is particularly preferred.

The proportion of the third lubricant relative to 100 parts by mass of the third shielding agent is, for example, 0.5 to 100 parts by mass, preferably 1 to 95 parts by mass, more preferably 10 to 90 parts by mass, even more preferably 30 to 80 parts by mass, and most preferably 50 to 70 parts by mass. In a case where the proportion of the lubricant is excessively low, it may be difficult to form a smooth intermediate layer. In a case where the proportion of the lubricant is excessively high, the intermediate layer may have low mechanical characteristics.

The intermediate layer may further contain other additives including, for example, the above-mentioned plasticizer, surfactant, pH adjusting agent, coloring agent, and/or the after-mentioned diluent. In the intermediate layer, the total proportion of other additives relative to 100 parts by mass of the third shielding agent is, for example, not more than 10 parts by mass, and is preferably not more than 5 parts by mass (for example, 0.1 to 5 parts by mass).

The intermediate layer may be in the form of a single layer (or may have a single-layered structure) or may be in the form of a multilayer (or may have a laminated structure). The intermediate layer in the form of a multilayer may be composed of layers each different in the proportion of the lubricant.

The intermediate layer has an average thickness (for a laminated structure, an average thickness of a total thickness) of, for example, 10 to 50 μm, preferably 15 to 45 μm, more preferably 20 to 40 μm, and most preferably 25 to 35 μm. In a case where the thickness of the intermediate layer is excessively small (or thin), the intermediate layer may have low mechanical characteristics. In a case where the thickness of the intermediate layer is excessively large (or thick), the oral preparation may have a low formulation designability.

[Outer layer]

The coat may further contain an outer layer coating the controlled release layer. In particular, when a tablet containing the controlled release granule is formed, coating of the controlled release layer with the outer layer can improve the mechanical characteristics of the tablet and can make it possible to uniformly disperse the controlled release granule in the tablet.

The outer layer may contain a diluent (a first diluent) and/or a lubricant (a fourth lubricant).

The first diluent may include, for example, a saccharide (such as lactose, glucose, fructose, maltose, sucrose, white soft sugar, and powdered hydrogenated maltose starch syrup), a sugar alcohol (such as D-mannitol, D-sorbitol, erythritol, and xylitol), a cellulose (such as a crystalline cellulose, a microcrystalline cellulose, and a powdered cellulose), and a cellulose ether (such as a methylcellulose and an ethylcellulose). These diluents (or excipients) may be used alone or in combination of two or more. Among them, the sugar alcohol such as D-mannitol is preferred.

The fourth lubricant may include, a silicic acid compound (such as a talc, a light anhydrous silicic acid, a calcium silicate, a magnesium silicate, a synthetic aluminum silicate, and a magnesium aluminometasilicate), in addition to any lubricant exemplified in the first lubricant of the controlled release layer. These lubricants for the outer layer may be used alone or in combination of two or more. Among them, the silicic acid compound such as the talc is preferred.

The total proportion of the first diluent and the fourth lubricant in the outer layer may be not less than 50% by mass, and is, for example, not less than 70% by mass, preferably not less than 80% by mass, more preferably not less than 90% by mass; the total proportion is most preferably 100% by mass (or the outer layer substantially contains only the first diluent and/or the fourth lubricant). In a case where the proportion of the diluent and/or the lubricant is excessively low, the coating with the outer layer may have a low effect.

The outer layer may further contain other additives including, for example, the above-mentioned binder, plasticizer, surfactant, pH adjusting agent, coloring agent, and/or the after-mentioned disintegrator. The total proportion of other additives relative to 100 parts by mass of the total of the first diluent and the fourth lubricant is, for example, not more than 10 parts by mass, and is preferably not more than 5 parts by mass (for example, 0.1 to 5 parts by mass).

The outer layer may contain (or may be formed with) only the first diluent or fourth lubricant. In a case where the outer layer contains only the lubricant, the lubricant does not necessarily need to form a layer, and may partially be attached to the surface of the coat. In this description and claims, an embodiment in which the lubricant is partially attached to the surface of the coat is also included in one embodiment of the outer layer.

The outer layer has an average thickness of, for example, 1 to 15 μm, preferably 1.5 to 10 μm, more preferably 2 to 8 μm, and most preferably 3 to 5 μm. In a case where the thickness of the outer layer is excessively small (or thin), the coating with the outer layer may have a low effect. In a case where the thickness of the outer layer is excessively large (or thick), the oral preparation may have a low formulation designability.

[Characteristics of coat]

The coat may be formed with the controlled release layer alone. As mentioned above, from the viewpoint that the reduction of the efficacy can be prevented, the coat preferably contains the controlled release layer and the shielding layer in combination; from the viewpoint that the mechanical characteristics of the coat can be improved, the coat more preferably contains the controlled release layer, the shielding layer, and the intermediate layer in combination; from the viewpoint that the tablet (in particular, an orally disintegrating tablet) can be more efficiently produced, the coat most preferably contains the controlled release layer, the shielding layer, the intermediate layer, and the outer layer in combination.

The coat may contain an esomeprazole compound. From the viewpoint that the formulation designability can be improved, it is preferred that the coat be free from an esomeprazole compound. In a case where the coat is in the form of a multilayer (or the coat is formed with a plurality of layers), it is preferred that all layers be free from an esomeprazole compound as an active ingredient.

The proportion of the coat in the controlled release granule may be not less than 50% by mass (in particular, not less than 80% by mass), and is, for example, 80 to 98% by mass, preferably 82 to 97% by mass, more preferably 85 to 95% by mass, and most preferably 87 to 93% by mass. In a case where the proportion of the coat is excessively low, the oral preparation may have a low controlled release ability. In a case where the proportion of the coat is excessively high, the oral preparation may have a low formulation designability or a low efficacy.

The coat has an average thickness of, for example, 30 to 130 $\mu$m, preferably 50 to 120 $\mu$m, more preferably 55 to 110 $\mu$m, and most preferably 60 to 105 $\mu$m. In a case where the thickness of the coat is excessively small (or thin), the oral preparation may have a low controlled release ability. In a case where the thickness is excessively large (or thick), the oral preparation may have a low formulation designability or a low efficacy.

[Oral preparation]

The oral preparation of the present disclosure contains the controlled release granule. The controlled release granule has a median particle diameter ($d_{50}$) of not more than 350 $\mu$m, for example, 180 to 350 $\mu$m, preferably 200 to 330 $\mu$m, more preferably 230 to 300 $\mu$m, even more preferably 240 to 280 $\mu$m, and most preferably 250 to 260 $\mu$m. In a case where the median particle diameter of the controlled release granule is excessively large, the oral preparation may have a low formulation designability.

The sharpness index of the particle size distribution of the controlled release granule may be selected from a range of about 1 to 5, and is, for example, 1 to 3, preferably 1 to 2, more preferably 1 to 1.5, even more preferably 1 to 1.3, and most preferably 1 to 1.2. In a case where the sharpness index is excessively large, the oral preparation may have a low formulation designability.

The dosage form of the oral preparation of the present disclosure may include, but should not be limited to, liquid dosage forms or semi-solid dosage forms. From the viewpoint of a significantly improved effect on the formulation designability, the solid dosage forms are preferred.

The solid dosage forms may include, for example, powdered preparations, powders, granulated preparations (such as granules and fine granules), pills, tablets (such as sublingual tablets, orally disintegrating tablets, troches, and chewable tablets), capsules (such as hard capsules, soft capsules, and microcapsules), and film-like preparations (or sheet-like preparations). Among them, preferred are tablets of which a controlled release ability is difficult to maintain due to a controlled release layer easily broken in a production process, in terms of a significant improvement effect by the present disclosure; particularly preferred are orally disintegrating tablets (OD tablets), which tend to have a reduced comfortability of taking.

An OD tablet of the present disclosure may be obtained by tableting a composition containing the controlled release granule; the OD tablet may further contain a diluent (a second diluent) and a disintegrator in addition to the controlled release granule.

As the second diluent, there may be used any diluent exemplified in the first diluent of the outer layer. The diluents (or excipients) may be used alone or in combination of two or more. Among the diluents, the sugar alcohol such as D-mannitol, the cellulose such as the crystalline cellulose, and the cellulose ether such as the ethylcellulose are preferred.

The proportion of the second diluent relative to 100 parts by mass of controlled release granule is, for example, 10 to 200 parts by mass, preferably 30 to 100 parts by mass, more preferably 40 to 80 parts by mass, and most preferably 50 to 75 parts by mass. In a case where the proportion of the second diluent is excessively low, the OD tablet may have low mechanical characteristics. In a case where the proportion of the second diluent is excessively high, the OD tablet may have a low disintegration.

The disintegrator may include, for example, a polysaccharide [a starch such as a corn starch, a potato starch, a pregelatinized starch, a partially pregelatinized starch, an oxidized starch, a dextrin, a cyclodextrin, a hydroxypropyl starch, a carboxymethyl starch, and a sodium carboxymethyl starch; a cellulose ether such as a carmellose, a carmellose sodium, a carmellose calcium, a croscarmellose sodium, and a low substituted hydroxypropylcellulose (L-HPC); an agar, a carrageenan, a gum acacia (or a gum arabic), an alginic acid, a sodium alginate, a propylene glycol alginate, a guar gum, a locust bean gum, a gum acacia (or a gum arabic), a tragacanth gum, a pullulan, a xanthan gum, a hyaluronic acid, a pectin, and a sodium chondroitin sulfate], a protein (such as a gelatin, a casein, and a soybean protein), a polyvinylpyrrolidone compound [such as a polyvinylpyrrolidone (a povidone), a vinylpyrrolidone copolymer (a copolyvidone), and a crospovidone], a silicic acid compound (such as a talc, a light anhydrous silicic acid, a calcium silicate, a magnesium silicate, a synthetic aluminum silicate, and a magnesium aluminometasilicate), and a mineral (such as a bentonite, a synthetic hydrotalcite, and a kaolin). These disintegrators may be used alone or in combination of two or more. Among these disintegrators, the starch such as the corn starch, the polyvinylpyrrolidone compound such as the crospovidone, and the silicic acid compound such as the light anhydrous silicic acid are preferred, and the starch and the crospovidone are particularly preferred.

The proportion of the disintegrator relative to 100 parts by mass of the controlled release granule is, for example, 3 to 100 parts by mass, preferably 5 to 50 parts by mass, more preferably 15 to 40 parts by mass, and most preferably 20 to 35 parts by mass. In a case where the proportion of the disintegrator is excessively low, the OD tablet may have a low disintegration. In a case where the proportion of the disintegrator is excessively high, the OD tablet may have low mechanical characteristics.

The OD tablet of the present disclosure may further contain other additives. Examples of other additives may include the above-mentioned plasticizer, surfactant, pH adjusting agent, shielding agent, binder, lubricant, and coloring agent and may additionally include a sweetening agent or a corrigent (such as aspartame, acesulfame potassium, sucralose, ascorbic acid, stevia, menthol, a crude glycyrrhiza extract, and a simple syrup), an aromatic agent or a perfume (such as menthol and a ginger oil), an algefacient or refrigerant, an antioxidant [such as dibutylhydroxytoluene (BHT), propyl gallate, butylhydroxyanisol (BHA), tocopherol, and citric acid], an antiseptic agent or a preservative (such as sodium benzoate and a parahydroxybenzoic acid ester), a wetting agent, an antistatic agent, and a disintegrant aid. These additives may be used alone or in combination of two or more. Among them, widely used are the sweetening agent, the perfume, and others. The total proportion of other additives relative to 100 parts by mass of the controlled release granule is, for example, not more than 10 parts by mass, and is preferably not more than 5 parts by mass (for example, 0.1 to 5 parts by mass).

The OD tablet of the present disclosure has a tablet diameter of, for example, 3 to 20 mm, preferably 5 to 15 mm, more preferably 7 to 12 mm, and most preferably 8 to 11 mm.

[Method for producing oral preparation]

The oral preparation of the present disclosure is obtained by a production method including a spherical-core-forming step of forming a spherical core with an esomeprazole compound and a coating step of coating the resulting spherical core with a coat to give a controlled release granule.

In the spherical-core-forming step, a method of forming the spherical core may include a conventional granulation method such as spray granulation, tumbling granulation, agitation granulation, and granulation in liquid. In order to make the spherical diameter of the spherical core small and to avoid the disintegration into powder of the granule in the coating step, a spherical particle consisting of an esomeprazole compound and having a high sphericity is preferably used as the spherical core.

In the coating step, a method of coating the coat may include a conventional coating method according to the dosage form, for example, application, spraying, impregnation or immersion, pan coating, fluidized bed coating, tumbling coating, and tumbling fluidized coating. Among them, fluidized bed coating and tumbling fluidized coating are preferred, and tumbling fluidized coating is particularly preferred. In a case where the coat is in the form of a multilayer (or the coat is formed with a plurality of layers), the coat may be formed using the coating methods different for the respective layers or may be formed using the same coating method for all layers. From the viewpoint of the efficient production of the oral preparation, it is preferred to form all layers by the same coating method, and it is particularly preferred to form all layers by tumbling fluidized coating.

The thus-obtained controlled release granule may directly be used as powdered preparations, granulated preparations, or other preparations, or the controlled release granule may be used to form solid dosage forms such as tablets and capsules by a conventional method. The OD tablet of the present disclosure can be obtained by tableting a mixture containing the controlled release granule, a diluent, and a disintegrator by a conventional method. The tableting pressure is, for example, 4 to 20 kN, preferably 5 to 15 kN, and more preferably 6 to 13 kN. A portion of the diluent and a portion of the disintegrator may be mixed and granulated to be used in a pellet state. The pellet has a particle diameter of, for example, 50 to 150 $\mu$m, and preferably 70 to 120 $\mu$m. In the present disclosure, the controlled release layer can be prevented from breakage even when a pressure is applied to the controlled release granule in tableting. The OD tablet has a hardness of, for example, 30 to 100 N, preferably 40 to 90 N, and more preferably 50 to 80 N.

The OD tablet of the present disclosure may be packaged in a conventional packaging form. The conventional packaging form may include, for example, a film packaging (such as a film packaging sealed by heat sealing or other means), a strip packaging (SP), and a press through packaging (PTP). Among them, the PTP is preferred.

The PTP may further be packaged with an aluminum pillow. As a packaging form having a PTP packaged with an aluminum pillow, a conventional aluminum pillow packaging form may be used.

The aluminum pillow packaging may have therein a desiccant and/or an oxygen absorber in addition to the PTP. As the desiccant, there may be used a conventional desiccant. The desiccant may include, for example, a silica gel, calcium chloride, and a zeolite. As the oxygen absorber, there may be used a conventional oxygen absorber. The oxygen absorber may include, for example, an iron-based oxygen absorber and an organic oxygen absorber. Among them, the desiccant is preferred from the viewpoint of easy expression of the effects of the present invention, and the zeolite is particularly preferred.

EXAMPLES

The following examples are intended to describe the present invention in further detail and should by no means be interpreted as defining the scope of the present invention. The materials and evaluation methods used in the following examples are shown below. The weights of materials used are all expressed in solid weight.

[Materials used in granule A]

(Active ingredient)

Esomeprazole magnesium trihydrate A
Esomeprazole magnesium trihydrate B

(Binder)

Hydroxypropylcellulose: product name "HPC-M" manufactured by Nippon Soda Co., Ltd., free from silicon dioxide
Ethylcellulose: product name "ETHOCEL Standard 7 Premium" manufactured by THE DOW CHEMICAL COMPANY, viscosity: 6 to 8 mPa·s

(Shielding agent)

Talc: product name "MICRO ACE P-3" manufactured by NIPPON TALC Co., Ltd.
Titanium oxide: product name "Titanium Oxide FG" manufactured by Freund Corporation

(Lubricant)

Magnesium stearate: Grade "Vegetable (Taihei)" manufactured by TAIHEI CHEMICAL INDUSTRIAL CO., LTD.
Hydrated silicon dioxide: product name "Adsolider-102" manufactured by FUJI SILYSIA CHEMICAL LTD.

(Enteric polymer)

Methacrylic acid copolymer LD: product name "EUDRAGIT L30D-55" manufactured by Evonik Industries AG
Dried methacrylic acid copolymer LD: product name "EUDRAGIT L100-55" manufactured by Evonik Industries AG
Ethyl acrylate-methyl methacrylate copolymer dispersion: product name "EUDRAGIT NE30D" manufactured by Evonik Industries AG

(Plasticizer)

Triethyl citrate: product name "Citroflex 2 (SC-60)" manufactured by MORIMURA BROS., INC.

(Surfactant)

Glyceryl monostearate: product name "Glyceryl monostearate P-100 10K" manufactured by RIKEN VITAMIN Co., Ltd.
Polysorbate 80: product name "Polysorbate 80" manufactured by Sanyo Chemical Industries, Ltd.

(pH Adjusting agent)

Citric acid hydrate: manufactured by SATUMA KAKO CO., LTD

(Diluent)

D-Mannitol: product name "PEARLITOL 50C" manufactured by ROQUETTE

[Evaluation methods]

(Particle size distribution)

The particle size distribution was measured in a dry state on a volume basis with a laser diffraction particle size analyzer (product name "Mastersizer 3000" manufactured by Malvern Instruments Ltd., hereinafter which is referred to as a "laser diffraction measurement") to determine a median particle diameter ($d_{50}$) and a sharpness index.

(SEM image)

The SEM image was observed with a scanning electron microscope (product name "E-7800" manufactured by KEYENCE CORPORATION).

(Powder X-ray diffraction)

The powder X-ray diffraction (XRD) was measured with a benchtop powder X-ray diffraction instrument (product name "MiniFlex300" manufactured by Rigaku Corporation).

(Dissolution rate after tableting)

For the resulting OD tablets, the dissolution rate (n = 3) of esomeprazole magnesium hydrate at 120 minutes after the start of the test was determined in accordance with Dissolution Test in The Japanese Pharmacopoeia 16th Edition, General Tests. The apparatus used and measurement conditions are as follows.

<Apparatus used>

- Dissolution tester/RT-J2000 (manufactured by DAINIPPON SEIKI CO., LTD.)
- Ultra violet-visible spectrophotometer/UV-1800 type (manufactured by SHIMADZU CORPORATION)

<Measurement conditions>

- Dissolution medium: 1st fluid for dissolution test (pH 1.2)
- Volume of dissolution medium: 900 mL
- Rotation speed of paddle: 50 rpm

- Liquid temperature: 37°C
- Measurement wavelength (esomeprazole): 322 nm

Example 1

(Coating step of first intermediate layer)

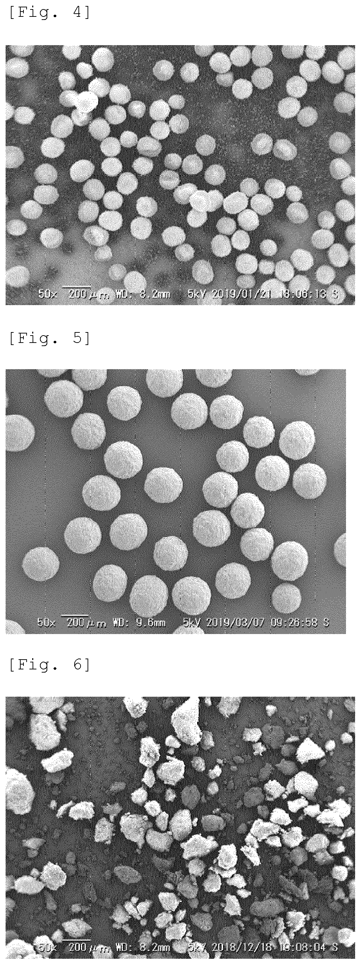

1) Esomeprazole magnesium trihydrate A (22.3 mg) and hydrated silicon dioxide (0.25 mg) were mixed, and the mixed powder was classified by a 100-mesh (100M, 150 μm) sieve and a 140M sieve (106 μm). The granular material which passed through the 100M sieve and remained on the 140M sieve was used as a spherical core of esomeprazole magnesium trihydrate A (in detail, a spherical particle of esomeprazole magnesium trihydrate A having a very small amount of hydrated silicon dioxide attached thereto). Fig. 3 is a SEM photograph of esomeprazole magnesium trihydrate A before classification, and Fig. 4 is a SEM photograph of a spherical core of esomeprazole magnesium trihydrate A after classification. The median particle diameter ($d_{50}$) and the sharpness index of the spherical core by laser diffraction measurement were 140 μm and 1.31, respectively.
2) The spherical core of esomeprazole magnesium trihydrate A was placed in a tumbling fluid bed granulator dryer (product name "Fluid bed granulator dryer FD-MP-01D" manufactured by Powrex Corporation).
3) Purified water (98 mg) and ethanol (60 mg) were mixed, and in the resulting liquid mixture was dissolved hydroxypropylcellulose (4 mg). Then, talc (24.8 mg) and magnesium stearate (1.6 mg) were dispersed in the resulting solution to give a dispersion 3.
4) Separately from the above-mentioned step, titanium oxide (6.6 mg) was dispersed in purified water (42 mg), and the resulting dispersion was filtered with a 200M sieve (75 μm) to give a filtrate 4.
5) The dispersion 3 and the filtrate 4 were mixed, and the spherical core was sprayed with the whole amount of the resulting liquid mixture to give a coated product.
6) Then, the coated product was dried.
7) The dried product and hydrated silicon dioxide (0.05 mg) were mixed, and the resulting mixed powder was classified by a 60M sieve and a 140M sieve. The granular material which passed through the 60M sieve (250 μm) and remained on the 140M sieve was used as an intermediate layer granule I.

(Coating step of second intermediate layer)

1) The intermediate layer granule I was placed in a tumbling fluid bed granulator dryer.
2) Purified water (98 mg) and ethanol (60 mg) were mixed, and in the resulting liquid mixture was dissolved hydroxypropylcellulose (4 mg). Then, talc (24.8 mg) and magnesium stearate (1.6 mg) were dispersed in the resulting solution to give a dispersion 2.
3) Separately from the above-mentioned step, titanium oxide (6.6 mg) was dispersed in purified water (42 mg), and the resulting dispersion was filtered with a 200M sieve to give a filtrate 3.
4) The dispersion 2 and the filtrate 3 were mixed, and the granule was sprayed with the whole amount of the resulting liquid mixture to give a coated product.
5) Then, the coated product was dried.
6) The dried product and hydrated silicon dioxide (0.05 mg) were mixed, and the resulting mixed powder was classified by a 60M sieve and a 140M sieve. The granular material which passed through the 60M sieve and remained on the 140M sieve was used as an intermediate layer granule II.

(Coating step of shielding layer)

1) The intermediate layer granule II was placed in a tumbling fluid bed granulator dryer.
2) Purified water (18 mg) and ethanol (42 mg) were mixed, and in the resulting liquid mixture was dissolved ethylcellulose (3 mg). Then, talc (9 mg) and magnesium stearate (4.5 mg) were dispersed in the resulting solution to give a dispersion 2.
3) The granule was sprayed with the whole amount of the dispersion 2 to give a coated product.
4) Then, the coated product was dried.

5) The dried product and hydrated silicon dioxide (0.05 mg) were mixed, and the resulting mixed powder was classified by a 60M sieve and a 100M sieve. The granular material which passed through the 60M sieve and remained on the 100M sieve was used as a shielding layer granule.

(Coating of enteric coating layer)

1) The shielding layer granule was placed in a tumbling fluid bed granulator dryer.
2) Citric acid hydrate (0.015 mg) was dissolved in purified water (60 mg), and the resulting solution and ethyl acrylate-methyl methacrylate copolymer dispersion (5.76 mg (solid weight)) were mixed to give a dispersion 2.
3) Polysorbate 80 (0.56 mg), glyceryl monostearate (5.6 mg), and triethyl citrate (11.52 mg) were dispersed in purified water (60 mg) to give a dispersion 3.
4) Methacrylic acid copolymer LD (57.6 mg), the dispersion 2, and the dispersion 3 were mixed. The resulting mixture was filtered with a 100M sieve to give a filtrate, and the granule was sprayed with the whole amount of the filtrate to give a coated product.
5) Then, the coated product was dried.
6) The dried product was classified by a 42M sieve (355 $\mu$m) and an 83M sieve (180 $\mu$m). The granular material which passed through the 42M sieve and remained on the 83M sieve was used as an enteric layer granule.

(Coating step of outer layer)

1) The enteric layer granule was placed in a tumbling fluid bed granulator dryer.
2) D-mannitol (10.16 mg) was dissolved in purified water (63.5 mg), and the granule was sprayed with the whole amount of the resulting solution to give a coated product.
3) Then, the coated product was dried.
4) The dried product was classified by a 42M sieve and an 83M sieve. The granule which passed through the 42M sieve and remained on the 83M sieve was used as an outer layer granule.

(Preparation of cured granule)

1) The outer layer granule was cured in a constant temperature and humidity chamber, and followed by drying.
2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 273 $\mu$m and 1.11, respectively. Fig. 5 is a SEM photograph of the resulting granule A as a controlled release granule.

Table 1 shows a composition of the resulting granule A.
[Table 1]

Table 1

| | | | |
|---|---|---|---|
| Intermediate layer granule I | Active ingredient | Esomeprazole magnesium trihydrate A | 22.3 mg |
| | Binder | Hydroxypropylcellulose | 4 mg |
| | Shielding agent | Talc | 24.8 mg |
| | Shielding agent | Titanium oxide | 6.6 mg |
| | Lubricant | Magnesium stearate | 1.6 mq |
| | Lubricant | Hydrated silicon dioxide | 0.3 mg |

(continued)

| | | | |
|---|---|---|---|
| Intermediate layer granule II | Binder | Hydroxypropylcellulose | 4 mg |
| | Shielding agent | Talc | 24.8 mg |
| | Shielding agent | Titanium oxide | 6.6 mg |
| | Lubricant | Magnesium stearate | 1.6 mg |
| | Lubricant | Hydrated silicon dioxide | 0.05 mg |
| Shielding layer granule | Binder | Ethylcellulose | 3 mg |
| | Shielding agent | Talc | 9 mg |
| | Lubricant | Magnesium stearate | 4.5 mg |
| | Lubricant | Hydrated silicon dioxide | 0.05 mg |
| Enteric layer granule | Enteric polymer | Methacrylic acid copolymer LD | 57.6 mg |
| | Enteric polymer | Ethyl acrylate-methyl methacrylate copolymer dispersion | 5.76 mg |
| | Plasticizer | Triethyl citrate | 11.52 mg |
| | Surfactant | Glyceryl monostearate | 5.6 mg |
| | Surfactant | Polysorbate 80 | 0.56 mg |
| | pH Adjusting agent | Citric acid hydrate | 0.015 mg |
| Outer layer granule | Diluent | D-Mannitol | 10.16 mg |

(Preparation of granule B)

1) D-mannitol (92.93 mg), crystalline cellulose (2.82 mg) and ethylcellulose (2.82 mg) as diluents, and light anhydrous silicic acid (1.41 mg) as a disintegrator were placed in a fluid bed granulator dryer and were mixed to give a mixed powder.
2) Corn starch (28.16 mg) and crospovidone (12.67 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized to give a granule B.

(Tableting step)

1) Using a diffusion mixer (product name "Bohle Container Mixer LM-20" manufactured by Hiroshima Metal & Machinery Co., Ltd.), the following were mixed: 204 mg of the granule A; 140.8 mg of the granule B; 20 mg of crystalline cellulose (product name "Ceolus KG-802" manufactured by Asahi Kasei Corp., average degree of polymerization: 200 to 300, loss on drying: 2 to 6%, bulk density: 0.13 to 0.23 g/cm$^3$) and 20 mg of ethylcellulose (ethylcellulose: product name "ETHOCEL Standard 7 Premium" manufactured by THE DOW CHEMICAL COMPANY) as diluents; 20 mg of crospovidone (product name "Kollidon CL-F", Type A manufactured by BASF Japan Ltd.) as a disintegrator; 2 mg of aspartame (manufactured by Ajinomoto Co., Inc.) as a sweetening agent; and 0.4 mg of peppermint micron (product name "Peppermint Micron H-81550" manufactured by TAKASAGO INTERNATIONAL CORPORATION) as a perfume. Furthermore, 1.2 mg of magnesium stearate as a lubricant was placed therein, and the contents were mixed to give a powder for tableting.
2) The powder for tableting was tableted by a single-punch tableting machine to give an OD tablet having a tablet diameter of 9.5 mm and a thickness of 5.4 mm.

Example 2

(Production of OD tablet)

An OD tablet having a tablet diameter of 9.5 mm and a thickness of 5.4 mm was obtained in the same manner as in Example 1 except that a spherical core of esomeprazole magnesium trihydrate A had a granularity and a sharpness index different from those of the spherical core used in the above-mentioned Example 1 and that the amount of ethyl acrylate-methyl methacrylate copolymer dispersion was changed to 2.88 mg (solid weight) and the amount of triethyl citrate was changed to 14.4 mg in coating of the enteric coating layer.

Example 3

(Coating step of first intermediate layer)

1) Esomeprazole magnesium trihydrate A (22.3 mg) having a granularity and a sharpness index different from those of the esomeprazole trihydrates A used in the above-mentioned Examples 1 and 2 and hydrated silicon dioxide (0.25 mg) were mixed, and the mixed powder was classified by a 100M sieve and a 140M sieve. The granular material which passed through the 100M sieve and remained on the 140M sieve was used as a spherical core of esomeprazole magnesium trihydrate A. The median particle diameter ($d_{50}$) and the sharpness index of the resulting spherical core by laser diffraction measurement were 147 μm and 1.24, respectively.
2) The spherical core of esomeprazole magnesium trihydrate A was placed in a tumbling fluid bed granulator dryer.
3) Purified water (98 mg) and ethanol (60 mg) were mixed, and in the resulting liquid mixture was dissolved hydroxypropylcellulose (4 mg). Then, talc (24.8 mg) and magnesium stearate (1.6 mg) were dispersed in the resulting solution to give a dispersion 3.
4) Separately from the above-mentioned step, titanium oxide (6.6 mg) was dispersed in purified water (42 mg), and the resulting dispersion was filtered with a 200M sieve to give a filtrate 4.
5) The dispersion 3 and the filtrate 4 were mixed, and the spherical core was sprayed with the whole amount of the resulting liquid mixture to give a coated product.
6) Then, the coated product was dried.
7) The dried product and hydrated silicon dioxide (0.05 mg) were mixed, and the resulting mixed powder was classified by a 60M sieve and a 140M sieve. The granular material which passed through the 60M sieve and remained on the 140M sieve was used as an intermediate layer granule I.

(Coating step of second intermediate layer)

1) The intermediate layer granule I was placed in a tumbling fluid bed granulator dryer.
2) Purified water (98 mg) and ethanol (60 mg) were mixed, and in the resulting liquid mixture was dissolved hydroxypropylcellulose (4 mg). Then, talc (24.8 mg) and magnesium stearate (1.6 mg) were dispersed in the resulting solution to give a dispersion 2.
3) Separately from the above-mentioned step, titanium oxide (6.6 mg) was dispersed in purified water (42 mg), and the resulting dispersion was filtered with a 200M sieve to give a filtrate 3.
4) The dispersion 2 and the filtrate 3 were mixed, and the granule was sprayed with the whole amount of the resulting liquid mixture to give a coated product.
5) Then, the coated product was dried.
6) The dried product and hydrated silicon dioxide (0.05 mg) were mixed, and the resulting mixed powder was classified by a 60M sieve and a 140M sieve. The granular material which passed through the 60M sieve and remained on the 140M sieve was used as an intermediate layer granule II.

(Coating step of shielding layer)

1) The intermediate layer granule II was placed in a tumbling fluid bed granulator dryer.
2) Purified water (140 mg) and ethanol (60 mg) were mixed, and in the resulting liquid mixture was dissolved hydroxypropylcellulose (10 mg). Then, talc (30 mg), magnesium stearate (1.5 mg), red ferric oxide (0.028 mg), and yellow ferric oxide (0.052 mg) were dispersed in the resulting solution to give a dispersion 2.

3) The granule was sprayed with the whole amount of the dispersion 2 to give a coated product.

4) Then, the coated product was dried.

5) The dried product and hydrated silicon dioxide (0.05 mg) were mixed, and the resulting mixed powder was classified by a 60M sieve and a 100M sieve. The granular material which passed through the 60M sieve and remained on the 100M sieve was used as a shielding layer granule.

(Coating step of enteric coating layer)

1) The shielding layer granule was placed in a tumbling fluid bed granulator dryer.

2) Citric acid hydrate (0.015 mg) was dissolved in purified water (60 mg), and the resulting solution and ethyl acrylate-methyl methacrylate copolymer dispersion (5.63 mg (solid weight)) were mixed to give a dispersion 2.

3) Polysorbate 80 (2.24 mg), glyceryl monostearate (5.6 mg), and triethyl citrate (11.26 mg) were dispersed in purified water (60 mg) to give a dispersion 3.

4) Methacrylic acid copolymer LD (56.28 mg), the dispersion 2, and the dispersion 3 were mixed. The resulting mixture was filtered with a 100M sieve to give a filtrate, and the granule was sprayed with the whole amount of the filtrate to give a coated product.

5) Then, the coated product was dried.

6) The dried product was classified by a 42M sieve and an 83M sieve. The granular material which passed through the 42M sieve and remained on the 83M sieve was used as an enteric layer granule.

(Coating step of outer layer)

1) The enteric layer granule was placed in a tumbling fluid bed granulator dryer.

2) D-mannitol (13.2 mg) was dissolved in purified water (82.5 mg), and the granule was sprayed with the whole amount of the resulting solution to give a coated product.

3) Then, the coated product was dried.

4) The dried product was classified by a 42M sieve and an 83M sieve. The granular material which passed through the 42M sieve and remained on the 83M sieve was used as an outer layer granule.

(Preparation of cured granule)

1) The outer layer granule was cured in constant temperature and humidity chamber, and followed by drying.

2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 283 $\mu$m and 1.32, respectively.

Table 2 shows a composition of the resulting granule A.

[Table 2]

Table 2

| | | | |
|---|---|---|---|
| Intermediate | Active ingredient | Esomeprazole magnesium trihydrate A | 22.3 mg |
| | Binder | Hydroxypropylcellulose | 4 mg |
| | Shielding agent | Talc | 24.8 mg |
| | Shielding agent | Titanium oxide | 6.6 mg |
| | Lubricant | Magnesium stearate | 1.6 mg |
| | Lubricant | Hydrated silicon dioxide | 0.3 mg |
| Intermediate layer granule II | Binder | Hydroxypropylcellulose | 4 mg |
| | Shielding agent | Talc | 24.8 mg |
| | Shielding agent | Titanium oxide | 6.6 mg |
| | Lubricant | Magnesium stearate | 1.6 mq |
| | Lubricant | Hydrated silicon dioxide | 0.05 mg |
| Shielding layer granule | Binder | Hydroxypropylcellulose | 10 mg |
| | Shielding agent | Talc | 30 mg |
| | Lubricant | Magnesium stearate | 1.5 mg |
| | Lubricant | Hydrated silicon dioxide | 0.05 mg |
| | Coloring agent | Red ferric oxide | 0.028 mg |
| | Coloring agent | Yellow ferric oxide | 0.052 mg |
| Enteric layer granule | Enteric polymer | Methacrylic acid copolymer LD | 56.28 mg |
| | Enteric polymer | Ethyl acrylate-methyl methacrylate copolymer dispersion | 5.63 mg |
| | Plasticizer | Triethyl citrate | 11.26 mg |
| | Surfactant | Glyceryl monostearate | 5.6 mg |
| | Surfactant | Polysorbate 80 | 2.24 mg |
| | pH Adjusting agent | Citric acid hydrate | 0.015 mg |
| Outer layer granule | Diluent | D-Mannitol | 13.2 mg |

(Preparation of granule B)

1) D-mannitol (77.44 mg), crystalline cellulose (2.26 mg) and ethylcellulose (2.26 mg) as diluents, and light anhydrous silicic acid (1.13 mg) as a disintegrator were placed in a fluid bed granulator dryer and were mixed to give a mixed powder.
2) Corn starch (22.56 mg) and crospovidone (10.15 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized to give a granule B.

(Tableting step)

1) Using a diffusion mixer, the following were mixed: 232 mg of the granule A; 112.8 mg of the granule B; 20 mg of crospovidone as a disintegrator; 32 mg of ethylcellulose as a diluent; 2 mg of aspartame as a sweetening agent;

and 0.4 mg of peppermint micron as a perfume. Furthermore, 1.2 mg of magnesium stearate as a lubricant was placed therein, and the contents were mixed to give a powder for tableting.

2) The powder for tableting was tableted by a single-punch tableting machine to give an OD tablet having a tablet diameter of 9.5 mm and a thickness of 5.4 mm.

Example 4

(Coating step of first intermediate layer)

1) Esomeprazole magnesium trihydrate A (22.3 mg) having a granularity and a sharpness index different from those of the esomeprazole trihydrates A used in the above-mentioned Examples 1 to 3 and hydrated silicon dioxide (0.25 mg) were mixed, and the mixed powder was classified by a 100M sieve and a 140M sieve. The granular material which passed through the 100M sieve and remained on the 140M sieve was used as a spherical core of esomeprazole magnesium trihydrate A. The median particle diameter ($d_{50}$) and the sharpness index of the resulting spherical core by laser diffraction measurement were 136 $\mu$m and 1.31, respectively.

2) The spherical core of esomeprazole magnesium trihydrate A was placed in a tumbling fluid bed granulator dryer.

3) Purified water (98 mg) and ethanol (60 mg) were mixed, and in the resulting liquid mixture was dissolved hydroxypropylcellulose (4 mg). Then, talc (12 mg) and magnesium stearate (12 mg) were dispersed in the resulting solution to give a dispersion 3.

4) Separately from the above-mentioned step, titanium oxide (6.6 mg) was dispersed in purified water (42 mg), and the resulting dispersion was filtered with a 200M sieve to give a filtrate 4.

5) The dispersion 3 and the filtrate 4 were mixed, and the spherical core was sprayed with the whole amount of the resulting liquid mixture to give a coated product.

6) Then, the coated product was dried.

7) The dried product and hydrated silicon dioxide (0.05 mg) were mixed, and the resulting mixed powder was classified by a 60M sieve and a 140M sieve. The granular material which passed through the 60M sieve and remained on the 140M sieve was used as an intermediate layer granule I.

(Coating step of second intermediate layer)

1) The intermediate layer granule I was placed in a tumbling fluid bed granulator dryer.

2) Purified water (98 mg) and ethanol (60 mg) were mixed, and in the resulting liquid mixture was dissolved hydroxypropylcellulose (4 mg). Then, talc (12 mg) and magnesium stearate (12 mg) were dispersed in the resulting solution to give a dispersion 2.

3) Separately from the above-mentioned step, titanium oxide (6.6 mg) was dispersed in purified water (42 mg), and the resulting dispersion was filtered with a 200M sieve to give a filtrate 3.

4) The dispersion 2 and the filtrate 3 were mixed, and the granule was sprayed with the whole amount of the resulting liquid mixture to give a coated product.

5) Then, the coated product was dried.

6) The dried product and hydrated silicon dioxide (0.05 mg) were mixed, and the resulting mixed powder was classified by a 60M sieve and a 140M sieve. The granular material which passed through the 60M sieve and remained on the 140M sieve was used as an intermediate layer granule II.

(Coating step of shielding layer)

1) The intermediate layer granule II was placed in a tumbling fluid bed granulator dryer.

2) Purified water (42 mg) and ethanol (18 mg) were mixed, and in the resulting liquid mixture was dissolved hydroxypropylcellulose (3 mg). Then, talc (9 mg) and magnesium stearate (4.5 mg) were dispersed in the resulting solution to give a dispersion 2.

3) The granule was sprayed with the whole amount of the dispersion 2 to give a coated product.

4) Then, the coated product was dried.

5) The dried product and hydrated silicon dioxide (0.05 mg) were mixed, and the resulting mixed powder was classified by a 60M sieve and a 100M sieve. The granular material which passed through the 60M sieve and remained on the 100M sieve was used as a shielding layer granule.

(Coating step of enteric coating layer)

1) The shielding layer granule was placed in a tumbling fluid bed granulator dryer.
2) Citric acid hydrate (0.015 mg) was dissolved in purified water (60 mg), and the resulting solution and ethyl acrylate-methyl methacrylate copolymer dispersion (5.76 mg (solid weight)) were mixed to give a dispersion 2.
3) Polysorbate 80 (0.56 mg), glyceryl monostearate (5.6 mg), and triethyl citrate (11.52 mg) were dispersed in purified water (60 mg) to give a dispersion 3.
4) Methacrylic acid copolymer LD (57.6 mg), the dispersion 2, and the dispersion 3 were mixed. The resulting mixture was filtered with a 100M sieve to give a filtrate, and the granule was sprayed with the whole amount of the filtrate to give a coated product.
5) Then, the coated product was dried.
6) The dried product was classified by a 42M sieve and an 83M sieve. The granular material which passed through the 42M sieve and remained on the 83M sieve was used as an enteric layer granule.

(Coating step of outer layer)

1) The enteric layer granule was placed in a tumbling fluid bed granulator dryer.
2) D-mannitol (10.16 mg) was dissolved in purified water (63.5 mg), and the granule was sprayed with the whole amount of the resulting solution to give a coated product.
3) Then, the coated product was dried.
4) The dried product was classified by a 42M sieve and an 83M sieve. The granule which passed through the 42M sieve and remained on the 83M sieve was used as an outer layer granule.

(Preparation of cured granule)

1) The outer layer granule was cured in a constant temperature and humidity chamber, and followed by drying.
2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 252 $\mu$m and 1.19, respectively.

Table 3 shows a composition of the resulting granule A.

[Table 3]

Table 3

|  |  |  |  |
| --- | --- | --- | --- |
|  | Active ingredient | Esomeprazole magnesium trihydrate A | 22.3 mg |
|  | Binder | Hydroxypropylcellulose | 4 mg |
| Intermediate | Shielding agent | Talc | 12 mg |
|  | Shielding agent | Titanium oxide | 6.6 mg |
|  | Lubricant | Magnesium stearate | 12 mg |
|  | Lubricant | Hydrated silicon dioxide | 0.3 mg |

(continued)

| | Binder | Hydroxypropylcellulose | 4 mg |
|---|---|---|---|
| Intermediate layer granule II | Shielding agent | Talc | 12 mg |
| | Shielding agent | Titanium oxide | 6.6 mg |
| | Lubricant | Magnesium stearate | 12 mg |
| | Lubricant | Hydrated silicon dioxide | 0.05 mg |
| Shielding layer granule | Binder | Hydroxypropylcellulose | 3 mg |
| | Shielding agent | Talc | 9 mg |
| | Lubricant | Magnesium stearate | 4.5 mg |
| | Lubricant | Hydrated silicon dioxide | 0.05 mg |
| Enteric layer granule | Enteric polymer | Methacrylic acid copolymer LD | 57.6 mg |
| | Enteric polymer | Ethyl acrylate-methyl methacrylate copolymer dispersion | 5.76 mg |
| | Plasticizer | Triethyl citrate | 11.52 mg |
| | Surfactant | Glyceryl monostearate | 5.6 mg |
| | Surfactant | Polysorbate 80 | 0.56 mg |
| | pH Adjusting agent | Citric acid hydrate | 0.015 mg |
| Outer layer granule | Diluent | D-Mannitol | 10.16 mg |

(Preparation of granule B)

1) D-mannitol (94.11 mg), crystalline cellulose (2.91 mg) and ethylcellulose (2.91 mg) as diluents, and light anhydrous silicic acid (1.46 mg) as a disintegrator were placed in a fluid bed granulator dryer and were mixed to give a mixed powder.
2) Corn starch (29.12 mg) and crospovidone (13.1 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized to give a granule B.

(Tableting step)

1) Using a diffusion mixer, the following were mixed: 199.2 mg of the granule A; 145.6 mg of the granule B; 20 mg of crospovidone as a disintegrator; 20 mg of crystalline cellulose and 20 mg of ethylcellulose as diluents; 2 mg of aspartame as a sweetening agent; and 0.4 mg of peppermint micron as a perfume. Furthermore, 1.2 mg of magnesium stearate as a lubricant was placed therein, and the contents were mixed to give a powder for tableting.
2) The powder for tableting was tableted by a single-punch tableting machine to give an OD tablet having a tablet diameter of 9.5 mm and a thickness of 5.4 mm.

Example 5

(Coating step of enteric coating layer)

1) The shielding layer granule obtained in Example 4 was placed in a tumbling fluid bed granulator dryer.

2) Citric acid hydrate (0.047 mg) was dissolved in purified water (186 mg), and the resulting solution and ethyl acrylate-methyl methacrylate copolymer dispersion (17.86 mg (solid weight)) were mixed to give a dispersion 2.

3) Polysorbate 80 (1.74 mg), glyceryl monostearate (17.36 mg), and triethyl citrate (35.71 mg) were dispersed in purified water (186 mg) to give a dispersion 3.

4) Methacrylic acid copolymer LD (178.6 mg), the dispersion 2, and the dispersion 3 were mixed. The resulting mixture was filtered with a 100M sieve to give a filtrate, and the granule was sprayed with the whole amount of the filtrate to give a coated product.

5) Then, the coated product was dried.

6) The dried product was classified by a 42M sieve and an 83M sieve. The granular material which passed through the 42M sieve and remained on the 83M sieve was used as an enteric layer granule.

(Coating step of outer layer)

1) The enteric layer granule was placed in a tumbling fluid bed granulator dryer.

2) D-mannitol (10.78 mg) was dissolved in purified water (67.35 mg), and the granule was sprayed with the whole amount of the resulting solution to give a coated product.

3) Then, the coated product was dried.

4) The dried product was classified by a 42M sieve and an 83M sieve. The granular material which passed through the 42M sieve and remained on the 83M sieve was used as an outer layer granule.

(Preparation of cured granule)

1) The outer layer granule was cured in a constant temperature and humidity chamber, and followed by drying.

2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 321 $\mu$m and 1.15, respectively.

Table 4 shows a composition of the resulting granule A.

[Table 4]

Table 4

|  |  |  |  |
| --- | --- | --- | --- |
| Intermediate | Active ingredient | Esomeprazole magnesium trihydrate A | 22.3 mg |
|  | Binder | Hydroxypropylcellulose | 4 mg |
|  | Shielding agent | Talc | 12 mg |
|  | Shielding agent | Titanium oxide | 6.6 mg |
|  | Lubricant | Magnesium stearate | 12 mg |
|  | Lubricant | Hydrated silicon dioxide | 0.3 mg |
| Intermediate layer granule II | Binder | Hydroxypropylcellulose | 4 mg |
|  | Shielding agent | Talc | 12 mg |
|  | Shielding agent | Titanium oxide | 6.6 mg |
|  | Lubricant | Magnesium stearate | 12 mg |
|  | Lubricant | Hydrated silicon dioxide | 0.05 mg |

(continued)

| Shielding layer Granule | Binder | Hydroxypropylcellulose | 3 mg |
|---|---|---|---|
| | Shielding agent | Talc | 9 mg |
| | Lubricant | Magnesium stearate | 4.5 mg |
| | Lubricant | Hydrated silicon dioxide | 0.05 mg |
| Enteric layer granule | Enteric polymer | Methacrylic acid copolymer LD | 178.6 mg |
| | Enteric polymer | Ethyl acrylate-methyl methacrylate copolymer dispersion | 17.86 mg |
| | Plasticizer | Triethyl citrate | 35.71 mg |
| | Surfactant | Glyceryl monostearate | 17.36 mg |
| | Surfactant | Polysorbate 80 | 1.74 mg |
| | pH Adjusting agent | Citric acid hydrate | 0.047 mg |
| Outer layer granule | Diluent | D-Mannitol | 10.78 mg |

(Preparation of granule B)

1) D-mannitol (171.5 mg), crystalline cellulose (5.20 mg) and ethylcellulose (5.20 mg) as diluents, and light anhydrous silicic acid (2.60 mg) as a disintegrator were placed in a fluid bed granulator dryer and were mixed to give a mixed powder.
2) Corn starch (51.97 mg) and crospovidone B (23.39 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized to give a granule B.

(Tableting step)

1) Using a diffusion mixer, the following were mixed: 370 mg of the granule A; 259.84 mg of the granule B; 36 mg of crospovidone as a disintegrator; 36 mg of crystalline cellulose and 36 mg of ethylcellulose as diluents; 3.6 mg of aspartame as a sweetening agent; and 0.7 mg of peppermint micron as a perfume. Furthermore, 2.16 mg of magnesium stearate as a lubricant was placed therein, and the contents were mixed to give a powder for tableting.
2) The powder for tableting was tableted by a single-punch tableting machine to give an OD tablet having a tablet diameter of 12.0 mm.

Example 6

(Coating step of first intermediate layer)

1) The same esomeprazole magnesium trihydrate A (22.3 mg) as that used in Example 4 and hydrated silicon dioxide (0.25 mg) were mixed, and the mixed powder was classified by a 100M sieve and a 140M sieve. The granular material which passed through the 100M sieve and remained on the 140M sieve was used as a spherical core of esomeprazole magnesium trihydrate A.
2) The spherical core of esomeprazole magnesium trihydrate A was placed in a tumbling fluid bed granulator dryer.
3) Purified water (84 mg) and ethanol (51.75 mg) were mixed, and in the resulting liquid mixture was dissolved hydroxypropylcellulose (3.45 mg). Then, talc (10.35 mg) and magnesium stearate (10.25 mg) were dispersed in the resulting solution to give a dispersion 3.

4) Separately from the above-mentioned step, titanium oxide (5.7 mg) was dispersed in purified water (36.75 mg), and the resulting dispersion was filtered with a 200M sieve to give a filtrate 4.

5) The dispersion 3 and the filtrate 4 were mixed, and the spherical core was sprayed with the whole amount of the resulting liquid mixture to give a coated product.

6) Then, the coated product was dried.

7) The dried product and hydrated silicon dioxide (0.07 mg) were mixed, and the resulting mixed powder was classified by a 60M sieve and a 140M sieve. The granular material which passed through the 60M sieve and remained on the 140M sieve was used as an intermediate layer granule I.

(Coating step of second intermediate layer)

1) The intermediate layer granule I was placed in a tumbling fluid bed granulator dryer.

2) Purified water (84 mg) and ethanol (51.75 mg) were mixed, and in the resulting liquid mixture was dissolved hydroxypropylcellulose (3.45 mg). Then, talc (10.35 mg) and magnesium stearate (10.35 mg) were dispersed in the resulting solution to give a dispersion 2.

3) Separately from the above-mentioned step, titanium oxide (5.7 mg) was dispersed in purified water (36.75 mg), and the resulting dispersion was filtered with a 200M sieve to give a filtrate 3.

4) The dispersion 2 and the filtrate 3 were mixed, and the granule was sprayed with the whole amount of the resulting liquid mixture to give a coated product.

5) Then, the coated product was dried.

6) The dried product and hydrated silicon dioxide (0.02 mg) were mixed, and the resulting mixed powder was classified by a 60M sieve and a 100M sieve. The granular material which passed through the 60M sieve and remained on the 100M sieve was used as an intermediate layer granule II.

(Coating step of shielding layer)

1) The intermediate layer granule II was placed in a tumbling fluid bed granulator dryer.

2) Purified water (42 mg) and ethanol (18 mg) were mixed, and in the resulting liquid mixture was dissolved hydroxypropylcellulose (3 mg). Then, talc (9 mg) and magnesium stearate (4.5 mg) were dispersed in the resulting solution to give a dispersion 2.

3) The granule was sprayed with the whole amount of the dispersion 2 to give a coated product.

4) Then, the coated product was dried.

5) The dried product and hydrated silicon dioxide (0.02 mg) were mixed, and the resulting mixed powder was classified by a 60M sieve and a 100M sieve. The granular material which passed through the 60M sieve and remained on the 100M sieve was used as a shielding layer granule.

(Coating of first enteric coating layer)

1) The shielding layer granule was placed in a tumbling fluid bed granulator dryer.

2) Purified water (242.97 mg) and ethanol (364.49 mg) were mixed. A portion of the resulting liquid mixture was taken out, and dried methacrylic acid copolymer LD (40.32 mg) was dissolved in the portion to give a solution 2.

3) In the residual portion of the liquid mixture, Polysorbate 80 (4.03 mg), triethyl citrate (12.1 mg), and talc (12.1 mg) were dispersed, and the resulting dispersion was filtered with a 200M sieve to give a filtrate 3.

4) The solution 2 and the filtrate 3 were mixed, and the granule was sprayed with the whole amount of the resulting liquid mixture to give a coated product.

5) Then, the coated product was dried.

6) The dried product and hydrated silicon dioxide (0.02 mg) were mixed, and the resulting mixed powder was classified by a 42M sieve and an 83M sieve. The granular material which passed through the 42M sieve and remained on the 83M sieve was used as an enteric layer granule I.

(Coating of second enteric coating layer)

1) The enteric layer granule I was placed in a tumbling fluid bed granulator dryer.
2) Purified water (242.97 mg) and ethanol (364.49 mg) were mixed. A portion of the resulting liquid mixture was taken out, and dried methacrylic acid copolymer LD (40.32 mg) was dissolved in the portion to give a solution 2.
3) In the residual portion of the liquid mixture, Polysorbate 80 (4.03 mg), triethyl citrate (12.1 mg), and talc (12.1 mg) were dispersed, and the resulting dispersion was filtered with a 200M sieve to give a filtrate 3.
4) The solution 2 and the filtrate 3 were mixed, and the granule was sprayed with the whole amount of the resulting liquid mixture to give a coated product.
5) Then, the coated product was dried.
6) The dried product and hydrated silicon dioxide (0.02 mg) were mixed, and the resulting mixed powder was classified by a 42M sieve and an 83M sieve. The granular material which passed through the 42M sieve and remained on the 83M sieve was used as an enteric layer granule II.

(Preparation of cured granule)

1) The enteric layer granule II and talc (4.70 mg) were mixed to prepare an outer layer granule having talc attached to the surface of the granule. The resulting outer layer granule was cured in a constant temperature and humidity chamber, and followed by drying.
2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 293 $\mu$m and 1.27, respectively.

Table 5 shows a composition of the resulting granule A.

[Table 5]

Table 5

| | | | |
|---|---|---|---|
| Intermediate | Active ingredient | Esomeprazole magnesium trihydrate A | 22.3 mg |
| | Binder | Hydroxypropylcellulose | 3.45 mg |
| | Shielding agent | Talc | 10.35 mg |
| | Shielding agent | Titanium oxide | 5.7 mg |
| | Lubricant | Magnesium stearate | 10.25 mg |
| | Lubricant | Hydrated silicon dioxide | 0.32 mg |
| Intermediate layer granule II | Binder | Hydroxypropylcellulose | 3.45 mg |
| | Shielding agent | Talc | 10.35 mg |
| | Shielding agent | Titanium oxide | 5.7 mg |
| | Lubricant | Magnesium stearate | 10.35 mg |
| | Lubricant | Hydrated silicon dioxide | 0.02 mg |
| Shielding layer granule | Binder | Hydroxypropylcellulose | 3 mg |
| | Shielding agent | Talc | 9 mg |
| | Lubricant | Magnesium stearate | 4.5 mg |
| | Lubricant | Hydrated silicon dioxide | 0.02 mg |

(continued)

| | | | |
|---|---|---|---|
| Enteric layer granule I | Enteric polymer | Dried methacrylic acid copolymer LD | 40.32 mg |
| | Plasticizer | Triethyl citrate | 12.1 mg |
| | Shielding agent | Talc | 12.1 mg |
| | Surfactant | Polysorbate 80 | 4.03 mg |
| | Lubricant | Hydrated silicon dioxide | 0.02 mg |
| Enteric layer granule II | Enteric polymer | Dried methacrylic acid copolymer LD | 40.32 mg |
| | Plasticizer | Triethyl citrate | 12.1 mg |
| | Shielding agent | Talc | 12.1 mg |
| | Surfactant | Polysorbate 80 | 4.03 mg |
| | Lubricant | Hydrated silicon dioxide | 0.02 mg |
| Outer layer granule | Lubricant | Talc | 4.70 mg |

(Preparation of granule B)

1) D-mannitol (116.56 mg), crystalline cellulose (3.53 mg) and ethylcellulose (3.53 mg) as diluents, and light anhydrous silicic acid (1.77 mg) as a disintegrator were placed in a fluid bed granulator dryer and were mixed to give a mixed powder.
2) Corn starch (35.32 mg) and crospovidone (15.89 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized by a 30M sieve to give a granule B.

(Tableting step)

1) Using a diffusion mixer, the following were mixed: 240.2 mg of the granule A; 176.6 mg of the granule B; 20 mg of crystalline cellulose (product name "Ceolus KG-802" manufactured by Asahi Kasei Corp., average degree of polymerization: 200 to 300, loss on drying: 2 to 6%, bulk density: 0.13 to 0.23 g/cm$^3$) and 20 mg of ethylcellulose (ethylcellulose: product name "ETHOCEL Standard 7 Premium" manufactured by THE DOW CHEMICAL COMPANY) as diluents; 20 mg of crospovidone (product name "Kollidon CL-F", Type A manufactured by BASF Japan Ltd.) as a disintegrator; 2 mg of aspartame (manufactured by Ajinomoto Co., Inc.) as a sweetening agent; and 0.4 mg of peppermint micron (product name "Peppermint Micron H-81550" manufactured by TAKASAGO INTERNATIONAL CORPORATION) as a perfume. Furthermore, 1.2 mg of magnesium stearate as a lubricant was placed therein, and the contents were mixed to give a powder for tableting.
2) The powder for tableting was tableted by a rotary tableting machine to give an OD tablet having a tablet diameter of 9.5 mm and a thickness of 6.2 mm.

Comparative Example 1

(Preparation of extruded granule of esomeprazole magnesium hydrate)

1) Esomeprazole magnesium trihydrate B (22.3 mg) was placed in a high-speed agitation granulator.
2) Purified water (3.35 mg) and ethanol (3.35 mg) were mixed to give a liquid mixture, the whole amount of the liquid mixture was added to esomeprazole magnesium trihydrate B, and the resulting mixture was kneaded.
3) The kneaded product was extruded and sieved with a 30M sieve.

4) Then, the resulting product was dried.

5) The dried product was classified by a 60M sieve and a 140M sieve. The granular material which passed through the 60M sieve and remained on the 140M sieve was used as an extruded granule of esomeprazole magnesium hydrate B. Fig. 6 is a SEM image of the resulting extruded granule.

(Coating step of first intermediate layer)

1) The extruded granule of esomeprazole magnesium hydrate B was placed in a tumbling fluid bed granulator dryer.

2) Purified water (98 mg) and ethanol (60 mg) were mixed, and in the resulting liquid mixture was dissolved hydroxypropylcellulose (4 mg). Then, 24.8 mg and magnesium stearate (1.6 mg) were dispersed in the resulting solution to give a dispersion 2.

3) Separately from the above-mentioned step, titanium oxide (6.6 mg) was dispersed in purified water (42 mg), and the resulting dispersion was filtered with a 200M sieve to give a filtrate 3.

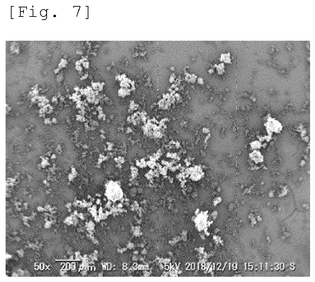

4) The dispersion 2 and the filtrate 3 were mixed, and the granule was sprayed with the whole amount of the resulting liquid mixture. The granule was disintegrated into powder at the time of 15% coating relative to the granule, and thus the coating was discontinued. Fig. 7 is a SEM image of the coated granule in a state disintegrated into powder.

Table 6 shows the evaluation results of the OD tablets obtained in Examples 1 to 6 and Comparative Example 1.

[Table 6]

Table 6

| | Spherical core | Laser diffraction measurement | | Proportion of coat in granule (% by mass) | Laser diffraction measurement | | Dissolution rate |
|---|---|---|---|---|---|---|---|
| | | $d_{50}$ of spherical core ($\mu$m) | Sharpness index | | $d_{50}$ of granule A ($\mu$m) | Sharpness index | (%) |
| Example 1 | Drug-containing spherical particle | 140 | 1.31 | 89.1 | 273 | 1.11 | 0.0 |
| Example 2 | | 145 | 1.22 | 89.1 | 270 | 1.19 | 0.7 |
| Example 3 | | 147 | 1.24 | 90.4 | 283 | 1.32 | 0.2 |
| Example 4 | | 136 | 1.31 | 88.8 | 252 | 1.19 | 0.3 |
| Example 5 | | 136 | 1.31 | 94.0 | 321 | 1.15 | 0.2 |
| Example 6 | | 136 | 1.31 | 90.7 | 293 | 1.27 | 0.4 |
| Comparative Example 1 | Extruded granule | - | - | - | - | - | - |

As apparent from the results of Table 6, the OD tablets obtained in Examples had a low tablet dissolution rate.

Reference Example 1

(Coating step of first intermediate layer)

1) The same esomeprazole magnesium trihydrate A (22.3 mg) as that used in Example 4 and hydrated silicon dioxide (0.25 mg) were mixed, and the mixed powder was classified by a 100M sieve and a 140M sieve. The granular material which passed through the 100M sieve and remained on the 140M sieve was used as a spherical core of esomeprazole magnesium trihydrate A.

2) The spherical core of esomeprazole magnesium trihydrate A was placed in a tumbling fluid bed granulator dryer.

3) Purified water (84 mg) and ethanol (51.75 mg) were mixed, and in the resulting liquid mixture was dissolved hydroxypropylcellulose (4 mg). Then, talc (12 mg) and magnesium stearate (12 mg) were dispersed in the resulting solution to give a dispersion 3.

4) Separately from the above-mentioned step, titanium oxide (6.6 mg) was dispersed in purified water (36.75 mg), and the resulting dispersion was filtered with a 200M sieve to give a filtrate 4.

5) The dispersion 3 and the filtrate 4 were mixed, and the spherical core was sprayed with the whole amount of the resulting liquid mixture to give a coated product.

6) Then, the coated product was dried.

7) The dried product and hydrated silicon dioxide (0.07 mg) were mixed, and the resulting mixed powder was classified by a 60M sieve and a 140M sieve. The granular material which passed through the 60M sieve and remained on the 140M sieve was used as an intermediate layer granule I.

(Coating step of second intermediate layer)

1) The intermediate layer granule I was placed in a tumbling fluid bed granulator dryer.

2) Purified water (84 mg) and ethanol (51.75 mg) were mixed, and in the resulting liquid mixture was dissolved hydroxypropylcellulose (4 mg). Then, talc (12 mg) and magnesium stearate (12 mg) were dispersed in the resulting solution to give a dispersion 2.

3) Separately from the above-mentioned step, titanium oxide (6.6 mg) was dispersed in purified water (36.75 mg), and the resulting dispersion was filtered with a 200M sieve to give a filtrate 3.

4) The dispersion 2 and the filtrate 3 were mixed, and the granule was sprayed with the whole amount of the resulting liquid mixture to give a coated product.

5) Then, the coated product was dried.

6) The dried product and hydrated silicon dioxide (0.02 mg) were mixed, and the resulting mixed powder was classified by a 60M sieve and a 100M sieve. The granular material which passed through the 60M sieve and remained on the 100M sieve was used as an intermediate layer granule II.

(Coating step of shielding layer)

1) The intermediate layer granule II was placed in a tumbling fluid bed granulator dryer.

2) Purified water (42 mg) and ethanol (18 mg) were mixed, and in the resulting liquid mixture was dissolved hydroxypropylcellulose (3 mg). Then, talc (9 mg) and magnesium stearate (4.5 mg) were dispersed in the resulting solution to give a dispersion 2.

3) The granule was sprayed with the whole amount of the dispersion 2 to give a coated product.

4) Then, the coated product was dried.

5) The dried product and hydrated silicon dioxide (0.02 mg) were mixed, and the resulting mixed powder was classified by a 60M sieve and a 100M sieve. The granular material which passed through the 60M sieve and remained on the 100M sieve was used as a shielding layer granule.

(Coating of enteric coating layer)

1) The shielding layer granule was placed in a tumbling fluid bed granulator dryer.

2) Purified water (485.94 mg) and ethanol (728.98 mg) were mixed. A portion of the resulting liquid mixture was taken out, and dried methacrylic acid copolymer LD (80.64 mg) was dissolved in the portion to give a solution 2.

3) In the residual portion of the liquid mixture, triethyl citrate (24.19 mg) and talc (24.19 mg) were dispersed, and the resulting dispersion was filtered with a 200M sieve to give a filtrate 3.

4) The solution 2 and the filtrate 3 were mixed, and the granule was sprayed with the whole amount of the resulting liquid mixture to give a coated product.

5) Then, the coated product was dried.

6) The dried product and hydrated silicon dioxide (0.02 mg) were mixed, and the resulting mixed powder was classified by a 42M sieve and an 83M sieve. The granular material which passed through the 42M sieve and remained on the 83M sieve was used as an enteric layer granule.

(Preparation of cured granule)

1) The enteric layer granule and talc (4.83 mg) were mixed to prepare an outer layer granule having talc attached to the surface of the granule. The resulting mixed powder was cured in a constant temperature and humidity chamber, and followed by drying.

2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 277 μm and 1.32, respectively.

Table 7 shows a composition of the resulting granule A.

[Table 7]

Table 7

| | | | |
|---|---|---|---|
| Intermediate | Active ingredient | Esomeprazole magnesium trihydrate A | 22.3 mg |
| | Binder | Hydroxypropylcellulose | 4 mg |
| | Shielding agent | Talc | 12 mg |
| | Shielding agent | Titanium oxide | 6.6 mg |
| | Lubricant | Magnesium stearate | 12 mg |
| | Lubricant | Hydrated silicon dioxide | 0.32 mg |
| Intermediate layer granule II | Binder | Hydroxypropylcellulose | 4 mg |
| | Shielding agent | Talc | 12 mg |
| | Shielding agent | Titanium oxide | 6.6 mg |
| | Lubricant | Magnesium stearate | 12 mg |
| | Lubricant | Hydrated silicon dioxide | 0.02 mg |
| Shielding layer granule | Binder | Hydroxypropylcellulose | 3 mg |
| | Shielding agent | Talc | 9 mg |
| | Lubricant | Magnesium stearate | 4.5 mg |
| | Lubricant | Hydrated silicon dioxide | 0.02 mg |
| Enteric layer granule | Enteric polymer | Dried methacrylic acid copolymer LD | 80.64 mg |
| | Plasticizer | Triethyl citrate | 24.19 mg |
| | Shielding agent | Talc | 24.19 mg |
| | Lubricant | Hydrated silicon dioxide | 0.02 mg |
| Outer layer granule | Lubricant | Talc | 4.83 mg |

(Preparation of granule B)

1) D-mannitol (115.47 mg), crystalline cellulose (3.50 mg) and ethylcellulose (3.50 mg) as diluents, and light anhydrous silicic acid (1.75 mg) as a disintegrator were placed in a fluid bed granulator dryer and were mixed to give a mixed powder.

2) Corn starch (35.0 mg) and crospovidone (15.75 mg) as disintegrators were dispersed in purified water to give a dispersion 2.

3) The mixed powder was sprayed with the whole amount of the dispersion 2 to give a coated product.

4) Then, the coated product was dried to give a powder 4.

5) The powder 4 was sized by a 30M sieve to give a granule B.

(Tableting step)

1) Using a diffusion mixer, the following were mixed: 241.85 mg of the granule A; 175.0 mg of the granule B; 20 mg of crystalline cellulose (product name "Ceolus KG-802" manufactured by Asahi Kasei Corp., average degree of polymerization: 200 to 300, loss on drying: 2 to 6%, bulk density: 0.13 to 0.23 g/cm$^3$) and 20 mg of ethylcellulose (ethylcellulose: product name "ETHOCEL Standard 7 Premium" manufactured by THE DOW CHEMICAL COMPANY) as diluents; 20 mg of crospovidone (product name "Kollidon CL-F", Type A manufactured by BASF Japan Ltd.) as a disintegrator; 2 mg of aspartame (manufactured by Ajinomoto Co., Inc.) as a sweetening agent; and 0.4 mg of peppermint micron (product name "Peppermint Micron H-81550" manufactured by TAKASAGO INTERNATIONAL CORPORATION) as a perfume. Furthermore, 1.2 mg of magnesium stearate as a lubricant was placed therein, and the contents were mixed to give a powder for tableting.

2) The powder for tableting was tableted by a rotary tableting machine to give an OD tablet having a tablet diameter of 9.5 mm and a thickness of 6.2 mm.

Reference Example 2

(Coating step of first intermediate layer)

1) The same esomeprazole magnesium trihydrate A (22.3 mg) as that used in Example 4 and hydrated silicon dioxide (0.25 mg) were mixed, and the mixed powder was classified by a 100M sieve and a 140M sieve. The granular material which passed through the 100M sieve and remained on the 140M sieve was used as a spherical core of esomeprazole magnesium trihydrate A.

2) The spherical core of esomeprazole magnesium trihydrate A was placed in a tumbling fluid bed granulator dryer.

3) Purified water (84 mg) and ethanol (51.75 mg) were mixed, and in the resulting liquid mixture was dissolved hydroxypropylcellulose (3.48 mg). Then, talc (10.44 mg) and magnesium stearate (10.44 mg) were dispersed in the resulting solution to give a dispersion 3.

4) Separately from the above-mentioned step, titanium oxide (5.74 mg) was dispersed in purified water (36.75 mg), and the resulting dispersion was filtered with a 200M sieve to give a filtrate 4.

5) The dispersion 3 and the filtrate 4 were mixed, and the spherical core was sprayed with the whole amount of the resulting liquid mixture to give a coated product.

6) Then, the coated product was dried.

7) The dried product and hydrated silicon dioxide (0.07 mg) were mixed, and the resulting mixed powder was classified by a 60M sieve and a 140M sieve. The granular material which passed through the 60M sieve and remained on the 140M sieve was used as an intermediate layer granule I.

(Coating step of second intermediate layer)

1) The intermediate layer granule I was placed in a tumbling fluid bed granulator dryer.

2) Purified water (84 mg) and ethanol (51.75 mg) were mixed, and in the resulting liquid mixture was dissolved hydroxypropylcellulose (3.46 mg). Then, talc (10.44 mg) and magnesium stearate (10.44 mg) were dispersed in the resulting solution to give a dispersion 2.

3) Separately from the above-mentioned step, titanium oxide (5.74 mg) was dispersed in purified water (36.75 mg), and the resulting dispersion was filtered with a 200M sieve to give a filtrate 3.

4) The dispersion 2 and the filtrate 3 were mixed, and the granule was sprayed with the whole amount of the resulting liquid mixture to give a coated product.

5) Then, the coated product was dried.

6) The dried product and hydrated silicon dioxide (0.02 mg) were mixed, and the resulting mixed powder was classified by a 60M sieve and a 100M sieve. The granular material which passed through the 60M sieve and remained on the 100M sieve was used as an intermediate layer granule II.

(Coating step of shielding layer)

1) The intermediate layer granule II was placed in a tumbling fluid bed granulator dryer.
2) Purified water (42 mg) and ethanol (18 mg) were mixed, and in the resulting liquid mixture was dissolved hydroxypropylcellulose (3 mg). Then, talc (9 mg) and magnesium stearate (4.5 mg) were dispersed in the resulting solution to give a dispersion 2.
3) The granule was sprayed with the whole amount of the dispersion 2 to give a coated product.
4) Then, the coated product was dried.
5) The dried product and hydrated silicon dioxide (0.02 mg) were mixed, and the resulting mixed powder was classified by a 60M sieve and a 100M sieve. The granular material which passed through the 60M sieve and remained on the 100M sieve was used as a shielding layer granule.

(Coating of first enteric coating layer)

1) The shielding layer granule was placed in a tumbling fluid bed granulator dryer.
2) Purified water (277.68 mg) and ethanol (416.56 mg) were mixed. A portion of the resulting liquid mixture was taken out, and dried methacrylic acid copolymer LD (46.08 mg) was dissolved in the portion to give a solution 2.
3) In the residual portion of the liquid mixture, triethyl citrate (13.82 mg) and talc (13.82 mg) were dispersed, and the resulting dispersion was filtered with a 200M sieve to give a filtrate 3.
4) The solution 2 and the filtrate 3 were mixed, and the granule was sprayed with the whole amount of the resulting liquid mixture to give a coated product.
5) Then, the coated product was dried.
6) The dried product and hydrated silicon dioxide (0.02 mg) were mixed, and the resulting mixed powder was classified by a 42M sieve and an 83M sieve. The granular material which passed through the 42M sieve and remained on the 83M sieve was used as an enteric layer granule I.

(Coating of second enteric coating layer)

1) The enteric layer granule I was placed in a tumbling fluid bed granulator dryer.
2) Purified water (208.26 mg) and ethanol (312.42 mg) were mixed. A portion of the resulting liquid mixture was taken out, and dried methacrylic acid copolymer LD (34.56 mg) was dissolved in the portion to give a solution 2.
3) In the residual portion of the liquid mixture, Polysorbate 80 (3.46 mg), triethyl citrate (10.37 mg), and talc (10.37 mg) were dispersed, and the resulting dispersion was filtered with a 200M sieve to give a filtrate 3.
4) The solution 2 and the filtrate 3 were mixed, and the granule was sprayed with the whole amount of the resulting liquid mixture to give a coated product.
5) Then, the coated product was dried.
6) The dried product and hydrated silicon dioxide (0.02 mg) were mixed, and the resulting mixed powder was classified by a 42M sieve and an 83M sieve. The granular material which passed through the 42M sieve and remained on the 83M sieve was used as an enteric layer granule II.

(Preparation of cured granule)

1) The enteric layer granule II and talc (4.52 mg) were mixed to prepare an outer layer granule having talc attached to the surface of the granule. The resulting mixed powder was cured in a constant temperature and humidity chamber, and followed by drying.
2) The dried product was classified by a 42M sieve and an 83M sieve. The granule which passed through the 42M sieve and remained on the 83M sieve was used as a granule A. The median particle diameter ($d_{50}$) and the sharpness index of the resulting granule A by laser diffraction measurement were 287 $\mu$m and 1.19, respectively.

Table 8 shows a composition of the resulting granule A.

[Table 8]

Table 8

| | | | |
|---|---|---|---|
| Intermediate | Active ingredient | Esomeprazole magnesium trihydrate A | 22.3 mg |
| | Binder | Hydroxypropylcellulose | 3.48 mg |
| | Shielding agent | Talc | 10.44 mg |
| | Shielding agent | Titanium oxide | 5.74 mq |
| | Lubricant | Magnesium stearate | 10.44 mg |
| | Lubricant | Hydrated silicon dioxide | 0.32 mg |
| Intermediate layer granule II | Binder | Hydroxypropylcellulose | 3.48 mg |
| | Shielding agent | Talc | 10.44 mg |
| | Shielding agent | Titanium oxide | 5.74 mq |
| | Lubricant | Magnesium stearate | 10.44 mg |
| | Lubricant | Hydrated silicon dioxide | 0.02 mg |
| Shielding layer granule | Binder | Hydroxypropylcellulose | 3 mg |
| | Shielding agent | Talc | 9 mg |
| | Lubricant | Magnesium stearate | 4.5 mg |
| | Lubricant | Hydrated silicon dioxide | 0.02 mg |
| Enteric | Enteric polymer | Dried methacrylic acid copolymer LD | 46.08 mg |
| | Plasticizer | Triethyl citrate | 13.82 mg |
| | Shielding agent | Talc | 13.82 mg |
| | Lubricant | Hydrated silicon dioxide | 0.02 mg |
| Enteric layer granule II | Enteric polymer | Dried methacrylic acid copolymer LD | 34.56 mg |
| | Plasticizer | Triethyl citrate | 10.37 mg |
| | Shielding agent | Talc | 10.37 mg |
| | Surfactant | Polysorbate 80 | 3.46 mg |
| | Lubricant | Hydrated silicon dioxide | 0.02 mg |
| Outer layer granule | Lubricant | Talc | 4.52 mg |

(Preparation of granule B)

1) D-mannitol (119.33 mg), crystalline cellulose (3.62 mg) and ethylcellulose (3.62 mg) as diluents, and light anhydrous silicic acid (1.81 mg) as a disintegrator were placed in a fluid bed granulator dryer and were mixed to give a mixed powder.
2) Corn starch (36.16 mg) and crospovidone (16.27 mg) as disintegrators were dispersed in purified water to give a dispersion 2.
3) The mixed powder was sprayed with the whole amount of the dispersion 2 to give a coated product.
4) Then, the coated product was dried to give a powder 4.
5) The powder 4 was sized by a 30M sieve to give a granule B.

(Tableting step)

1) Using a diffusion mixer, the following were mixed: 236 mg of the granule A; 180.8 mg of the granule B; 20 mg of crystalline cellulose (product name "Ceolus KG-802" manufactured by Asahi Kasei Corp., average degree of polymerization: 200 to 300, loss on drying: 2 to 6%, bulk density: 0.13 to 0.23 g/cm$^3$) and 20 mg of ethylcellulose (ethylcellulose: product name "ETHOCEL Standard 7 Premium" manufactured by THE DOW CHEMICAL COMPANY) as diluents; 20 mg of crospovidone (product name "Kollidon CL-F", Type A manufactured by BASF Japan Ltd.) as a disintegrator; 2 mg of aspartame (manufactured by Ajinomoto Co., Inc.) as a sweetening agent; and 0.4 mg of peppermint micron (product name "Peppermint Micron H-81550" manufactured by TAKASAGO INTERNATIONAL CORPORATION) as a perfume. Furthermore, 1.2 mg of magnesium stearate as a lubricant was placed therein, and the contents were mixed to give a powder for tableting.
2) The powder for tableting was tableted by a rotary tableting machine to give an OD tablet having a tablet diameter of 9.5 mm and a thickness of 6.2 mm.

INDUSTRIAL APPLICABILITY

The oral preparation of the present disclosure can express the pharmacological activity while preventing the deactivation of the esomeprazole compound when orally administrated. Thus, the oral preparation can effectively be used as the following: a therapeutic agent for gastric ulcer, duodenal ulcer, stomal ulcer, reflux esophagitis, non-erosive reflux disease, or Zollinger-Ellison syndrome; a therapeutic agent for preventing recurrence of gastric ulcer or duodenal ulcer due to administration of a non-steroidal antiinflammatory drug; and an adjuvant therapeutic agent for eradication of Helicobacter pylori in gastric ulcer, duodenal ulcer, gastric MALT lymphoma, idiopathic thrombocytopenic purpura, or stomach following endoscopic treatment of early gastric cancer.

**Claims**

1. An oral preparation comprising a controlled release granule having a median particle diameter ($d_{50}$) of not more than 350 $\mu$m, wherein the controlled release granule comprises a spherical core having a median particle diameter ($d_{50}$) of not less than 50 $\mu$m and containing an esomeprazole compound, and a coat coating the spherical core and containing a controlled release layer.

2. The oral preparation according to claim 1, wherein the spherical core contains the esomeprazole compound at a proportion of not less than 50% by mass relative to the spherical core.

3. The oral preparation according to claim 1 or 2, wherein the spherical core is a spherical particle of the esomeprazole compound.

4. The oral preparation according to any one of claims 1 to 3, wherein the spherical core has a sphericity of not less than 0.6.

5. The oral preparation according to any one of claims 1 to 4, wherein the coat is in a form of a multilayer free from an esomeprazole compound.

6. The oral preparation according to claim 5, wherein the coat further contains a shielding layer interposed between the spherical core and the controlled release layer.

7. The oral preparation according to claim 6, wherein the coat further contains an intermediate layer interposed between the spherical core and the shielding layer.

8. The oral preparation according to any one of claims 1 to 7, wherein the controlled release layer is an enteric coating layer.

9. The oral preparation according to any one of claims 1 to 8, wherein a proportion of the coat is not less than 80% by mass relative to a whole amount of the preparation.

**10.** The oral preparation according to any one of claims 1 to 9, which is in a form of a tablet.

**11.** The oral preparation according to any one of claims 1 to 10, which further contains a disintegrator and is in a form of an orally disintegrating tablet.

**12.** A method for producing an oral preparation recited in any one of claims 1 to 11, which comprises:

a spherical-core-forming step of forming a spherical core containing an esomeprazole compound, and
a coating step of coating the resulting spherical core with a coat to give a controlled release granule.

**13.** A method for improving a controlled release ability and a formulation designability of an oral preparation, the method comprising:

adjusting a median particle diameter ($d_{50}$) of a controlled release granule contained in an oral preparation to not more than 350 $\mu$m, and
preparing the controlled release granule comprising a spherical core and a coat coating the spherical core, the spherical core having a median particle diameter ($d_{50}$) of not less than 50 $\mu$m and containing an esomeprazole compound, and the coat containing a controlled release layer.

**14.** A method of use of a controlled release granule for improving a controlled release ability and a formulation designability of an oral preparation, wherein the controlled release granule comprises a spherical core having a median particle diameter ($d_{50}$) of not less than 50 $\mu$m and containing an esomeprazole compound, and a coat coating the spherical core and containing a controlled release layer, and has a median particle diameter ($d_{50}$) of not more than 350 $\mu$m.

[Fig. 1]

[Fig. 2]

[Fig. 3]

[Fig. 4]

[Fig. 5]

[Fig. 6]

[Fig. 7]

## INTERNATIONAL SEARCH REPORT

| International application No. |
| --- |
| PCT/JP2021/002521 |

A. CLASSIFICATION OF SUBJECT MATTER
A61K 31/4439(2006.01)i; A61K 9/14(2006.01)i; A61K 9/20(2006.01)i; A61K 9/28(2006.01)i; A61P 1/04(2006.01)i
FI: A61K31/4439; A61K9/14; A61K9/20; A61K9/28; A61P1/04

According to International Patent Classification (IPC) or to both national classification and IPC

B. FIELDS SEARCHED

Minimum documentation searched (classification system followed by classification symbols)
A61K31/4439; A61K9/14; A61K9/20; A61K9/28; A61P1/04

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2004-522796 A (ASTRAZENECA AKTIEBOLAG) 29 July 2004 (2004-07-29) claims, paragraphs [0013]-[0014], [0038], [0040]-[0041], examples 1, 3 | 1, 2, 4-14<br>1-14 |
| X<br>Y | JP 2019-99567 A (NIPPON CHEMIPHAR CO., LTD.) 24 June 2019 (2019-06-24) claims, paragraphs [0009], [0014], example 1 | 1, 2, 4-14<br>1-14 |
| Y | US 2016/0101055 A1 (PHARMADAX INC.) 14 April 2016 (2016-04-14) claims 1, 6, 8, paragraphs [0011], [0021], examples 10-12 | 1-14 |
| Y | JP 2000-281564 A (TAKEDA PHARMACEUTICAL COMPANY LIMITED) 10 October 2000 (2000-10-10) claims, paragraphs [0004]-[0005] | 1-14 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 03 March 2021 (03.03.2021) | 23 March 2021 (23.03.2021) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**

International application No.

PCT/JP2021/002521

C (Continuation). DOCUMENTS CONSIDERED TO BE RELEVANT

| Category* | Citation of document, with indication, where appropriate, of the relevant passages | Relevant to claim No. |
|---|---|---|
| A | 丹羽敏幸ほか，エマルション溶媒拡散法を利用した粒子設計－直接打錠用球形結晶からコロイド様 DDS の開発まで－, FRAGRANCE JOURNAL, 1993, vol. 21, no. 4, pp. 63-69, ISSN 0288-9803, in particular, pp. 64-65, non-official translation (NIWA, Toshiyuki et al., "Particulate designs using the emulsion solvent diffusion method -From spherical crystals for direct compression to the development of colloidal DDS-") | 1-14 |

Form PCT/ISA/210 (continuation of second sheet) (January 2015)

**INTERNATIONAL SEARCH REPORT**

Information on patent family members

| International application No. |
| --- |
| PCT/JP2021/002521 |

| Patent Documents referred in the Report | Publication Date | Patent Family | Publication Date |
| --- | --- | --- | --- |
| JP 2004-522796 A | 29 Jul. 2004 | US 2004/0101565 A1<br>claims, paragraphs<br>[0012]-[0013],<br>[0066], [0068]-<br>[0069], examples 1, 3<br>WO 2002/072071 A1<br>EP 1370243 A1<br>BR 207827 A<br>CN 1507343 A<br>KR 10-2003-0081506 A<br>ES 2307722 T3 | |
| JP 2019-99567 A | 24 Jun. 2019 | (Family: none) | |
| US 2016/0101055 A1 | 14 Apr. 2016 | US 2017/0224655 A1 | |
| JP 2000-281564 A | 10 Oct. 2000 | WO 1999/059544 A2<br>claims, column 2,<br>line 33 to column 5,<br>line 2<br>EP 1121103 A2<br>DE 69934505 T2<br>CN 1311669 A<br>ES 2274625 T3<br>KR 10-0554924 B1<br>US 6328994 B1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3665334 B
- JP 4649001 B
- JP 3350054 B
- JP 2018118936 A